(19) Europäisches Patentamt
European Patent Office
Office européen des brevets

(11) EP 3 484 890 B1

(12) **EUROPEAN PATENT SPECIFICATION**

(45) Date of publication and mention of the grant of the patent:
**02.09.2020 Bulletin 2020/36**

(21) Application number: **17735607.8**

(22) Date of filing: **11.07.2017**

(51) Int Cl.:
***C07D 487/22*** (2006.01) ***C25B 1/00*** (2006.01)

(86) International application number:
**PCT/EP2017/067452**

(87) International publication number:
**WO 2018/011229 (18.01.2018 Gazette 2018/03)**

(54) **SELECTIVE PORPHYRIN-CATALYZED ELECTROCHEMICAL REDUCTION OF CO2 INTO CO, IN PARTICULAR IN WATER**

SELEKTIVE PORPHYRIN-KATALYSIERTE ELEKTROCHEMISCHE REDUKTION VON CO2 IN CO, INSBESONDERE IN WASSER

RÉDUCTION ÉLECTROCHIMIQUE SÉLECTIVE DE CO2 EN CO, EN PARTICULIER DANS L'EAU, CATALYSÉE PAR LA PORPHYRINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2016 EP 16305904**

(43) Date of publication of application:
**22.05.2019 Bulletin 2019/21**

(73) Proprietors:
- **Centre National de la Recherche Scientifique (CNRS)**
  **75016 Paris (FR)**
- **Université Paris Diderot Paris 7**
  **75013 Paris (FR)**

(72) Inventors:
- **ROBERT, Marc**
  **75019 Paris (FR)**
- **SAVEANT, Jean-Michel**
  **75013 Paris (FR)**
- **COSTENTIN, Cyrille**
  **Brookline, MA 02246 (US)**
- **TATIN, Arnaud**
  **75013 Paris (FR)**
- **AZCARATE, Iban**
  **92800 Puteaux (FR)**

(74) Representative: **Regimbeau**
**20, rue de Chazelles**
**75847 Paris Cedex 17 (FR)**

(56) References cited:
**WO-A1-2015/169763**

- **CYRILLE COSTENTIN ET AL: "Efficient and selective molecular catalyst for the CO 2 -to-CO electrochemical conversion in water", PROCEEDINGS OF THE NATIONAL ACADEMY OF SCIENCES, vol. 112, no. 22, 18 May 2015 (2015-05-18) , pages 6882-6886, XP055259645, US ISSN: 0027-8424, DOI: 10.1073/pnas.1507063112 cited in the application**
- **S. A. SYRBU ET AL: "Synthesis of tetraphenylporphins with reactive groups in the phenyl rings. 7.* salts of tetrakis(N,N,N-trimethylaminophenyl)porphins", CHEMISTRY OF HETEROCYCLIC COMPOUNDS, vol. 32, no. 5, 1 May 1996 (1996-05-01), pages 573-576, XP055308897, US ISSN: 0009-3122, DOI: 10.1007/BF01164788**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to water soluble porphyrins, their iron complexes, use thereof as catalysts for the selective electrochemical reduction of $CO_2$ into CO, electrochemical cells comprising them, and methods for reducing electrochemically $CO_2$ into CO using said complexes or said electrochemical cells, thereby producing CO or syngas.

Despite the increasingly frequent use of renewable energies to produce electricity avoiding concomitant production of $CO_2$, it is reasonable to consider that $CO_2$ emissions, in particular resulting from energy production, will remain high in the next decades. It thus appears necessary to find ways to capture $CO_2$ gas, either for storing or valorization purposes.

Indeed, $CO_2$ can also be seen, not as a waste, but on the contrary as a source of carbon. For example the promising production of synthetic fuels from $CO_2$ and water has been envisaged.

However, $CO_2$ exhibits low chemical reactivity: breaking one its C-O bonds requires an energy of 532 kJ/mol. Moreover, $CO_2$ electrochemical reduction to one electron occurs at a very negative potential, thus necessitating a high energy input, and leads to the formation of a highly energetic radical anion ($CO_2^{\bullet -}$). Catalysis thus appears mandatory in order to reduce $CO_2$ and drive the multi-electronic and multi-proton reduction process, in order to obtain thermodynamically stable molecules. In addition, direct electrochemical reduction of $CO_2$ at inert electrodes is poorly selective, yielding formic acid in water, while it yields a mixture of oxalate, formate and carbon monoxide in low-acidity solvents such as DMF. $CO_2$ electrochemical reduction thus requires catalytic activation in order to reduce the energy cost of processing, and increase the selectivity of the species formed in the reaction process.

Several low-oxidation state transition metal complexes have been proposed to serve as homogeneous catalyst for this reaction in non-aqueous solvents such as N,N'-dimethylformamide (DMF) or acetonitrile (see Chem. Soc. Rev. 2013, 42, 2423).

Among them, electrochemically generated $Fe^0$ porphyrin complexes have been shown to be good catalysts provided they are used in the presence of Bronsted or Lewis acids (see J. Am. Chem. Soc. 1996, 118, 1769; J. Phys. Chem. 1996, 100, 19981). More recent investigations have extended the range of Bronsted acids able to boost the catalysis of the $CO_2$-to-CO conversion by electrogenerated $Fe^0$-TPP without degrading the selectivity of the reaction. They have also provided a detailed analysis of the reaction mechanism (see J. Am. Chem. Soc. 2013, 135, 9023).

This is notably the case with phenol, which gave rise to the idea of installing prepositioned phenol groups in the catalyst molecules "CAT" and "FCAT" depicted below. The result was indeed a remarkably efficient and selective catalyst of the $CO_2$-to-CO conversion in particular in terms of catalytic Tafel plots (Turnover frequency vs. overpotential) with no degradation of the CO (vs. $H_2$) faradaic yield (see Science 2012, 338, 90; and Proc. Natl. Acad. Sci. U.S.A. 2014, 111, 14990-14994).

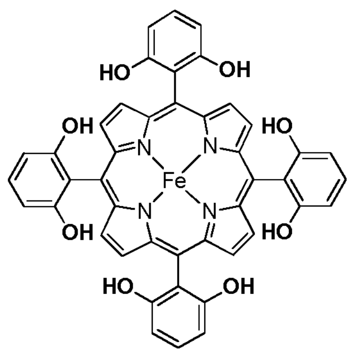

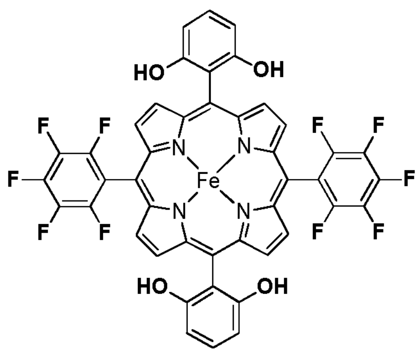

*Structure of the "CAT" catalyst (left) and "FCAT" catalyst (right)*

However, from the point of view of practical applications, the use of non-aqueous solvents is not optimal. It would be preferable indeed to be able to use water as the solvent, which would render more viable the $CO_2$-to-CO half-cell reaction as well as its association with a water-oxidation anode through a proton-exchange membrane for instance.

$CO_2$ is poorly soluble in water ($[CO_2]$ = 0.0383 M), and is partially converted ($K_{hydration} = 1.7 \times 10^{-3}$) into carbonic acid, $CO_3H_2$, which has a first ionization $pK_a$ of 3.6, i.e., an apparent $pK_a$ of 6.4. Because of these features, the $CO_2$-to-CO conversion is expected to be challenged by $H_2$ evolution from reduction of carbonic acid and/ or hydrated protons. Cobalt based catalyst cobalt tetrakis(4-trimethylammoniophenyl)porphyrin has been previously reported as an efficient catalyst for reduction of $CO_2$ into CO in water (Cao et al Acta Chimica Sinica 1986, 44, 220, pp 133-139). However, high $CO_2$ pressures are required to obtain high faradic efficiency and turnover numbers (TON) under reasonably low overpotential. A water-soluble water porphyrin has been developed - Fe-*p*-TMA - which proved very efficient for catalyzing the reduction of $CO_2$ into CO both in organic solvents and in water (see Proc. Natl. Acad. Sci. U.S.A., 2015, 112, 6882).

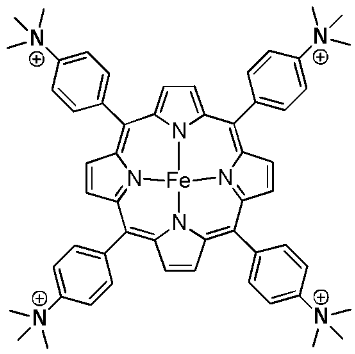

*Structure of Fe-p-TMA*

There is however still a need for catalysts for the selective and/or tunable electrochemical reduction of $CO_2$ into CO based on iron porphyrins with an even higher efficiency (i.e. high faradic yield, higher Turnover Number (TON) and Turnover Frequency (TOF)), higher selectivity and higher stability, while operating at low overpotential (in absolute value), and preferably under low $CO_2$ pressure, in particular in water. Moreover, it would be advantageous that the catalyst be efficient when operated homogeneously as well as when immobilized on an electrode surface.

## SUMMARY OF THE INVENTION

Applicants surprisingly found that specific water soluble iron porphyrins comprising four ortho anilinium substituents are particularly effective and selective catalysts for the electrochemical reduction of $CO_2$ into CO in water. The catalyst of the present invention exhibits high and tunable selectivity in organic solvents such as DMF as well as in water. Both homogeneous and heterogeneous catalytic systems are efficient, in particular at low overpotential and low $CO_2$ pressure.

Therefore, in a first aspect, the present invention relates to an iron complex of a porphyrin of formula (I) below:

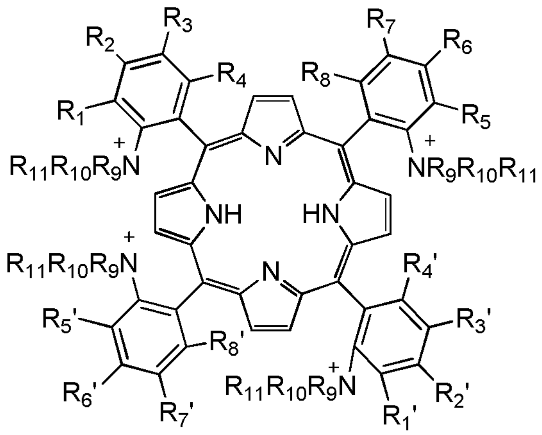

(I)

wherein $R_1$ to $R_8$ and $R_1$' to $R_8$' are independently selected from the group consisting of H, OH, F and $C_1$-$C_6$-alkyl, provided that at least 2 of $R_1$-$R_4$ are H, at least 2 of $R_{1'}$-$R_{4'}$ are H, at least 2 of $R_5$-$R_8$ are H, and at least 2 of $R_{5'}$-$R_{8'}$ are H, and

$R_9$, $R_{10}$ and $R_{11}$ are independently selected from a $C_1$-$C_4$-alkyl group,

and salts thereof.

In a second aspect, the present invention relates to the use of the iron complex of the invention as catalyst for the electrochemical reduction of $CO_2$ into CO, in particular in water.

In a third aspect, the present invention relates to a two-compartment electrochemical cell comprising at least:

- a cathodic compartment with a cathode and a cathodic electrolyte solution comprising a cathodic solvent and a cathodic supporting electrolyte, and the substrate $CO_2$,
- an anodic compartment with an anode and an anodic electrolyte solution comprising a solvent and an anodic

supporting electrolyte,
- a power supply providing the energy necessary to trigger the electrochemical reactions involving the substrate,

and further comprising the iron complex of the invention.
In a fourth aspect, the present invention relates to a method of reducing electrochemically $CO_2$ into CO using the electrochemical cell of the invention.

## DEFINITIONS

As used herein, the words "include," "comprise, "contain", and their variants, are intended to be non-limiting, such that recitation of items in a list is not to the exclusion of other like items that may also be useful in the compositions, devices and methods of this invention.
According to the present invention, an alkyl is understood to mean a linear or branched, saturated hydrocarbon chain. Examples of $C_1$-$C_6$ alkyl are methyl, ethyl, *n*-propyl, *i*-propyl, *n*-butyl, *s*-butyl, *t*-butyl, pentyl and n-hexyl.
According to the present invention, a $C_1$-$C_6$ alcohol is understood to mean a $C_1$-$C_6$ alkyl substituted by at least one hydroxyl group (OH group), preferably only one hydroxyl group. The $C_1$-$C_6$ alcohol may be linear or branched, and is saturated. Preferably, the $C_1$-$C_6$ alcohol is a $C_1$-$C_4$ alcohol. Examples of $C_1$-$C_4$ alcohol are hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 1-hydroxybutyl, 2-hydroxybutyl, 3-hydroxybutyl, 4-hydroxybutyl, 1-hydroxy-2-methylpropyl, 2-hydroxy-2-methylpropyl, 3-hydroxy-2-methylpropyl, 1-hydroxy-1-methylpropyl, 2-hydroxy-1-methylpropyl, 3-hydroxy-1-methylpropyl, (hydroxymethyl)-1-propyl, 1,2-dihydroxyethyl.
As used herein, "overpotential (η)" is understood as a potential difference between the apparent standard potential of the $CO_2$/CO couple ($E°_{CO2/CO}$) and the potential at which the reduction is experimentally observed (E), according to the following equation: $\eta = E°_{CO2/CO} - E$.
As used herein, the "TurnOver Number (TON)" represents the number of moles of substrate that a mole of active catalyst can convert.
As used herein, the "TurnOver Frequency (TOF)" refers to the turnover per unit of time: $TOF = \frac{TON}{t}$, with t representing the time of catalysis.
As used herein, the acronym NHE is understood as "Normal Hydrogen Electrode".
As used herein, the acronym SCE is understood as "Saturated Calomel Electrode". Electrolysis is for instance performed in an electrochemical cell, which typically comprises at least:

- an electrolyte solution comprising the solvent, a supporting electrolyte as a salt, and the substrate;
- a power supply providing the energy necessary to trigger the electrochemical reactions involving the substrate; and
- two electrodes, i.e. electrical conductors providing a physical interface between the electrical circuit and the solution.

As used herein, the "faradic yield of an electrochemical cell" aimed at producing CO (or $H_2$) gas through electrochemical reduction of $CO_2$ gas is the ratio of the amount of electrons (in Coulomb) used to produce CO (or $H_2$) gas relative to the amount of electrons (in Coulomb) furnished to the electrochemical system by the external electric source. The faradic yield is expressed in %.
According to the present invention, a "homogeneous catalyst" is a catalyst which is contained in the same phase as the reactants. In contrast, a "heterogeneous catalyst" is contained in a phase which differs from the phase of the reactants. Therefore, in the present invention, a "homogeneous catalyst" is soluble in the electrochemical cell solution. In particular, the homogeneous catalysts of the invention are soluble in water.
As used herein, "conductive polymers" are understood as organic polymers that conduct electricity. In particular, polyacetylene, polypyrrole, polyaniline, poly(p-phenylene vinylene) (PPV), poly(3-alkylthiophenes) and their copolymers are the main classes of conductive polymers. Examples of conductive polymers are polyfluorenes, polyphenylenes, polypyrenes, polyazulenes, polynaphthalenes, poly(pyrrole)s (PPY), polycarbazoles, polyindoles, polyazepines, polyanilines (PANI), poly(thiophene)s (PT), poly(3,4-ethylenedioxythiophene) (PEDOT), and poly(p-phenylene sulfide) (PPS). Preferred conductive polymers are polypyrrole, polyazepines, polyanilines or poly(3,4-ethylenedioxythiophene). It may also be a polymer in which a conductive material, such as carbon powder is embedded.
As used herein, an "ionomer" is understood as a polymer that comprises monomer units of both electrically neutral monomer units and a fraction of ionized monomer units (usually no more than 15 mole percent) covalently bound to the polymer backbone as lateral moieties. Most ionomers are copolymers of neutral segments and ionized units, said ionized units usually consisting of carboxylic acid groups or sulfonic acid groups. Preferred examples of ionomers are polystyrene sulfonates, and in particular Nafion®, a sulfonated tetrafluoroethylene based fluoropolymer-copolymer.

As used herein, a "fluoropolymer" is understood as a fluorocarbon based polymer with multiple carbon-fluorine bonds. In particular, a fluoropolymer results from a polymerization reaction using at least one type of fluorinated monomer. It is characterized by a high resistance to solvents, acids, and bases. Examples of suitable fluoropolymers are polyvinylfluoride and polyethylenetetrafluoroethylene.

As used herein, a "conductive material" is understood as a material that conducts electricity. Preferred examples of conductive materials are conductive carbon materials, and in particular carbon powder and carbon nanotubes.

As used herein, "syngas" is understood as a mixture of $H_2$ and CO gas, in any proportion. However, syngas does not encompass pure $H_2$ or pure CO gas.

As used herein, an "electrolyte solution" comprises a solvent and a supporting electrolyte, which is preferably a salt.

As used herein, an "aqueous mixture" of an organic solvent is a mixture of said organic solvent with water.

As used herein, a "buffer" or "buffering agent" is a weak acid or base used to maintain the acidity (pH) of a solution near a chosen value after the addition of another acid or base. That is, the function of a buffering agent is to prevent a rapid change in pH when acids or bases (such as $CO_2$) are added to the solution. Examples of buffers are phosphate buffer (such as $Na_2HPO_4$ or $Na_2HPO_4$ or mixtures thereof) and carbonate buffer (such as $KHCO_3$).

## DETAILED DESCRIPTION

### *Porphyrins*

First, the present invention concerns the iron complex of a porphyrin of formula (I) below:

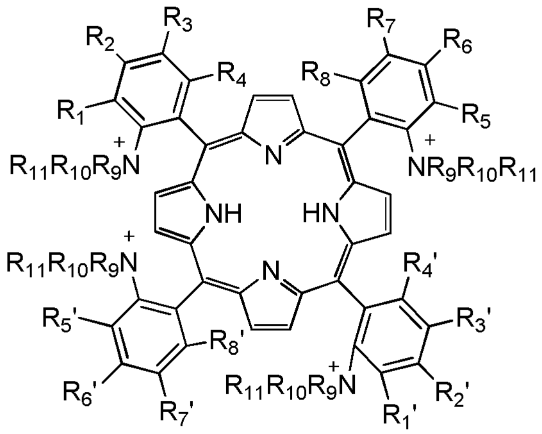

(I)

wherein $R_1$ to $R_8$ and $R_1$' to $R_8$' are independently selected from the group consisting of H, OH, F and $C_1$-$C_6$-alkyl, provided that at least 2 of $R_1$-$R_4$ are H, at least 2 of $R_{1'}$-$R_{4'}$ are H, at least 2 of $R_5$-$R_8$ are H, and at least 2 of $R_{5'}$-$R_{8'}$ are H, and

$R_9$, $R_{10}$ and $R_{11}$ are independently selected from a $C_1$-$C_4$-alkyl group,

and salts thereof.

The porphyrins of the invention may exist as different atropisomers. The present invention contemplates in particular all atropisomers, and mixtures thereof in all proportions.

Preferably, $R_9$, $R_{10}$ and $R_{11}$ are independently selected from a $C_1$-$C_2$-alkyl group, such as a methyl group. In a particular embodiment, $R_9$, $R_{10}$ and $R_{11}$ are identical, and in particular are a methyl group.

$R_1$ to $R_8$ and $R_1$' to $R_8$' are preferably independently selected from the group consisting of H, F and $C_1$-$C_6$-alkyl, preferably of H, F, even more preferably H and $C_1$-$C_4$-alkyl, most preferably H and methyl. Advantageously, at least 3 of $R_1$-$R_4$ are H, at least 3 of $R_{1'}$-$R_{4'}$ are H, at least 3 of $R_5$-$R_8$ are H, and at least 3 of $R_{5'}$-$R_{8'}$ are H.

In a particular embodiment, $R_1$, $R_2$, $R_3$, $R_4$, are respectively identical to $R_1$', $R_2$', $R_{3'}$, $R_4$'. In another particular embodiment, $R_5$, $R_6$, $R_7$, $R_8$, are respectively identical to $R_5$', $R_6$', $R_7$', $R_{8'}$.

In another particular embodiment, $R_1$, $R_2$, $R_3$, $R_4$, $R_5$, $R_6$, $R_7$, $R_8$, are respectively identical to $R_1$', $R_2$', $R_{3'}$, $R_4$', $R_5$', $R_6$', $R_7$', $R_8$'.

In another particular embodiment, $R_1$', $R_2$', $R_{3'}$, $R_4$' are respectively identical to $R_1$, $R_2$, $R_3$, $R_4$; $R_5$, $R_6$, $R_7$, $R_8$, are respectively identical to $R_1$, $R_2$, $R_3$, $R_4$; and $R_5$', $R_6$', $R_7$', $R_{8'}$ are respectively identical to $R_1$, $R_2$, $R_3$, $R_4$. In this embodiment, the porphyrin of the invention is of formula (Ia) :

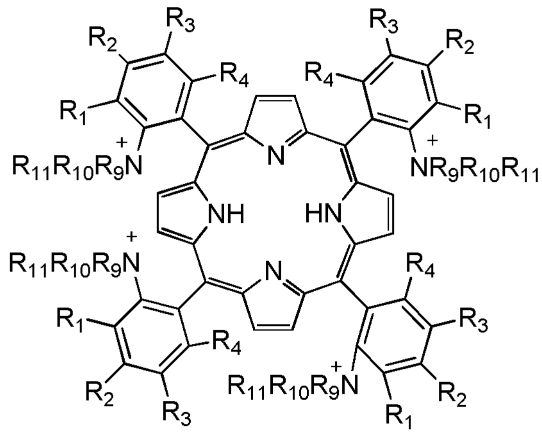

(Ia)

with $R_1$ to $R_4$ and $R_9$, $R_{10}$ and $R_{11}$ as defined above or below, or atropisomers thereof, and salts thereof, in particular a halogen salt thereof, such as a bromide or a chloride salt or a trifluoromethanesulfonate salt.

Most preferably, the porphyrin of the invention is of formula (Ib) :

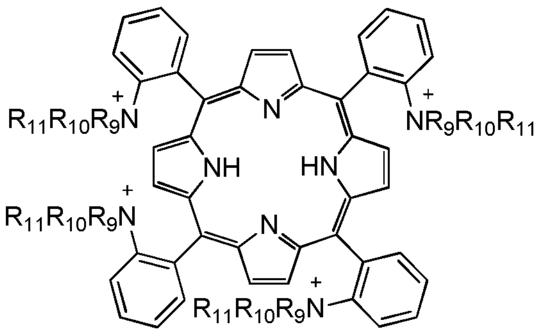

(Ib),

with $R_9$, $R_{10}$ and $R_{11}$ as defined above or below, or atropisomers thereof (in particular the α,β, α,β-atropisomer), and salts thereof, in particular a halogen salt thereof, such as a bromide or a chloride salt or a trifluoromethanesulfonate salt. Preferably, $R_9$, $R_{10}$ and $R_{11}$ are independently selected from a $C_1$-$C_2$-alkyl group, such as a methyl group. In a particular embodiment, $R_9$, $R_{10}$ and $R_{11}$ are identical.

For example, the iron complex of the invention is the iron complex of the following porphyrin:

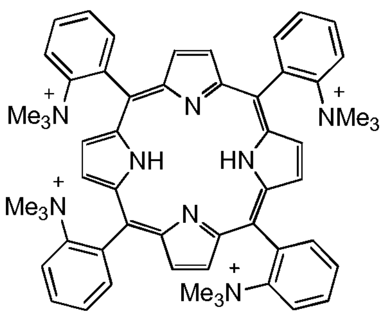

,

or atropisomers thereof, and salts thereof, in particular a halogen salt thereof, such as a bromide or a chloride salt thereof (in particular the tetrachloride salt). Of note, the iron complex of

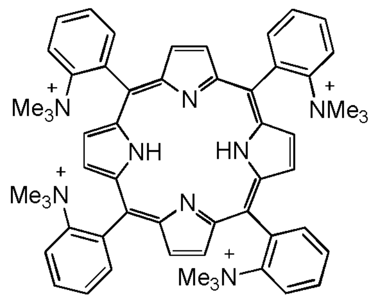

(especially as the trifluoromethanesulfonate salt) is referred to as Fe-o-TMA throughout the present description.
The metal transition complexes of the porphyrins of formula (I) are prepared according to methods well-known in the art (see in particular Costentin et al Proc. Natl. Acad. Sci. U.S.A. 2014, 111, 14990-14994).
The iron complexes of the invention may for instance be obtained from the corresponding tetra-*o*-nitro substituted porphyrins, which are then reduced to the corresponding tetra-*o*-amino substituted porphyrins. Said tetra-o-amino substituted porphyrins may then be alkylated (with $C_1$-$C_6$ alkyl groups or $C_1$-$C_6$ alkylaryl groups) to give the corresponding tetra-*o*-$NR_9R_{10}$ substituted porphyrins, which is then subjected to complexation with an iron salt such as $Fe(II)X_2$, with X representing a halogen such as bromide or chloride (preferably bromide), to yield the corresponding tetra-*o*-$NR_9R_{10}$ substituted porphyrins iron complex. Alternatively, complexation with iron may be carried out first under the same reaction conditions, and alkylation of the free amine may then be effected as described above. Finally, the neutral complex is alkylated (with $C_1$-$C_6$ alkyl groups or $C_1$-$C_6$ alkylaryl groups) to form the corresponding porphyrin of formula (I) containing four ortho-anilinium groups.
Of note, the complex is typically isolated as the Fe(III) complex, and more particularly as the Fe(III)Cl complex of the corresponding porphyrin of formula (I), where appropriate as a salt such as the chloride salt (in particular the tetrachloride salt). The active Fe(0) species (active Fe(0) complex for the reduction of $CO_2$ into CO) is generated *in situ* in the reaction medium (in particular in the electrochemical cell) from the Fe(III) complex. Therefore, in the present invention, $CO_2$ is reduced into CO by the porphyrin of formula (I) with Fe(0) (iron at the oxidation state of 0).
Therefore, the electrochemical reduction of $CO_2$ into CO with the catalyst of the invention typically involves the Fe(0), Fe(I), Fe(II) and Fe(III) complexes of the porphyrins of formula (I) as defined above.

***Use as Catalyst***

The present invention further relates to the use of the iron complex of the invention as catalyst for the electrochemical reduction of $CO_2$ into CO.
In other words, the present invention relates to a method of electrochemically reducing $CO_2$ into CO, comprising using the iron complex of the invention as catalyst.
In a particular embodiment, the iron complex of the invention is used for catalyzing the electrochemical reduction of $CO_2$ into CO in an organic solvent, such as such as DMF (dimethylformamide), ACN (acetonitrile), and aqueous mixtures thereof. In this embodiment, the electrolytes organic solvent further comprises a proton donor, advantageously with a pKa value in DMF of between 18 and 31, in particular selected from the group consisting of water ($H_2O$), trifluoroethanol, phenol and acetic acid, advantageously $H_2O$ or phenol. Advantageously, when a proton donor is used, said proton donor is used in a concentration of between 100 mM and 5M, preferably between 100 mM and 3M, even more preferably between 1M and 3M. In some embodiments, the electrolyte solvent is a solution of phenol or water in DMF, preferably 0.0-5.0 M solution of water or phenol in DMF, more preferably 0.0-2.5 M solution of water or phenol in DMF, even more preferably 1.0-2.0 M solution of water or phenol in DMF, and may contain additives (in particular supporting electrolytes) such as salts.
In another particular embodiment, the complex of the invention is used for catalyzing the electrochemical reduction of $CO_2$ into CO in water. In this case, the electrolyte solvent preferably consists in water and a supporting electrolyte. In other words, in this embodiment, the electrolyte solvent preferably does not contain any organic solvent, be it as co-solvent or proton-donor.
Depending on the experimental conditions, $H_2$ production may be promoted, and syngas may be produced.
In the present invention, the electrochemical reduction of $CO_2$ into CO is particularly selective. In particular, no formation of formic acid or formate is observed, while CO or syngas is produced.
Advantageously, in the present invention, the electrochemical reduction of $CO_2$ into CO is carried out at a $CO_2$ pressure of at least 1 bar such as between 1 and 30 bars. In a particular embodiment, the electrochemical reduction of $CO_2$ into CO is carried out at a $CO_2$ pressure of 1 bar (atmospheric pressure), preferably only in the cathodic compartment. In another embodiment, the electrochemical reduction of $CO_2$ into CO is carried out at a $CO_2$ pressure of more than 1

bar, for instance of between 2 and 30 bars, preferably only in the cathodic compartment.

In one embodiment, the iron complex of the invention is used as homogenous catalysts. In another embodiment, the iron complex of the invention is used as immobilized catalyst (for heterogeneous catalysis). In this embodiment, the catalyst is preferably immobilized on at least one electrode (preferably the cathode) using a binder. Therefore, in this embodiment, at least one electrode (preferably the cathode) comprises a composition comprising the catalyst and a binder. Advantageously, the composition is coated as a film on the electrode.

Advantageously, the binder is selected from the group consisting of conductive polymers, ionomers and/or fluoropolymers preferably a fluorinated polymer with sulfonic end groups such as a perfluorosulfonic acid. Preferred examples of binders are ionomers such as polystyrene sulfonates, and in particular Nafion®, a sulfonated tetrafluoroethylene based fluoropolymer-copolymer.

Advantageously, conductive materials are added to the binder to improve the conductivity of the system. Preferred examples of conductive materials are conductive carbon materials, and in particular carbon powder and carbon nanotubes.

***Electrochemical cell***

The present invention further relates to an electrochemical cell comprising at least:

- at least one electrolyte solution comprising a solvent and a supporting electrolyte such as a salt (preferably a mixture of alkali metal, such as hydroxide, carbonate, bicarbonate or chloride alkali metal salts), and the substrate $CO_2$,
- a power supply providing the energy necessary to trigger the electrochemical reactions involving the substrate, and
- two electrodes (an anode and a cathode),

and further comprising the iron complex of the invention.

In the electrochemical cell, the iron complex of the invention is used as catalyst for the electrochemical reduction of $CO_2$ into CO.

The particular and preferred embodiments of the porphyrin of formula (I) and the iron complex thereof are as described above.

The electrochemical cell of the invention comprises several compartments, in particular two compartments (the cathodic compartment comprising the cathode, and the anodic compartment comprising the anode), advantageously separated by a proton exchange membrane comprising or consisting of a ionomer or/and fluoropolymer, preferably a fluorinated polymer with sulfonic end groups such as a perfluorosulfonic acid.

In a preferred embodiment, the electrochemical cell of the invention comprises at least:

- a cathodic compartment with a cathode and a cathodic electrolyte solution comprising a cathodic solvent and a cathodic supporting electrolyte such as a salt, preferably a mixture of alkali metal (such as hydroxide, carbonate, bicarbonate or chloride alkali metal salts), and the substrate $CO_2$,
- an anodic compartment with an anode and an anodic electrolyte solution comprising a solvent and an anodic supporting electrolyte,
- a power supply providing the energy necessary to trigger the electrochemical reactions involving the substrate,

and further comprising the iron complex of a porphyrin of formula (I) as defined above. In a particular embodiment, the electrochemical cell of the invention is saturated with $CO_2$ gas, that is to say, both the atmosphere and the electrolyte solution are saturated with $CO_2$.

The electrochemical cell of the invention may be used as a closed system regarding $CO_2$ gas. This embodiment is of particular interest when the electrochemical cell of the invention is used to study the catalytic mechanism of the reduction of $CO_2$ into CO, as such a configuration allows for a tight control of gas evolution.

Conversely, the electrochemical cell of the invention may be used in an open environment, with a flow of $CO_2$ which saturates the electrolyte and solvent of the electrochemical cell of the invention. This configuration is particularly useful when industrial production of CO or syngas is sought for.

It is noteworthy that CO is typically not soluble in the electrolyte solution, so that it is collected directly as a gas.

Advantageously, $CO_2$ gas is present only in the cathodic compartment and the $CO_2$ pressure in the cathodic compartment of the electrochemical cell of the invention is of at least 1 bar. In a particular embodiment, the $CO_2$ pressure in the electrochemical cell of the invention is of 1 bar (atmospheric pressure), preferably only in the cathodic compartment. In another embodiment, the $CO_2$ pressure in the electrochemical cell of the invention (preferably only in the cathodic compartment) is of more than 1 bar, for instance of between 2 and 30 bars.

The power source may comprise one or more of power supplies (e.g., batteries and a photovoltaic cell).

Preferably, the anode is a carbon, iridium oxide, cobalt oxide, cobalt phosphate, stainless steel or platinum

electrode, for example it is a carbon, iridium oxide, cobalt oxide or platinum electrode. More preferably, the anode is an iridium oxide, cobalt oxide, cobalt phosphate or platinum electrode, and even more preferably it is an iridium oxide, cobalt oxide or platinum electrode.

Advantageously, the cathode is a carbon, stainless steel or mercury electrode. Preferably, it is a carbon electrode or a stainless steel electrode. More preferably, it is a carbon electrode such as a carbon crucible, carbon felt or carbon paper.

In a particular embodiment, the electrochemical cell further comprises a third electrode, preferably a reference electrode such as a standard calomel electrode or a silver chloride electrode.

The solvent of the electrolyte solutions is preferably selected from DMF (dimethylformamide), ACN (acetonitrile), water ($H_2O$) or mixtures thereof. When the solvent comprises DMF (dimethylformamide) and/or ACN (acetonitrile), said solvent advantageously further comprises a proton donor, preferably with a pKa value in DMF of between 18 and 31, in particular selected from the group consisting of water ($H_2O$), trifluoroethanol, phenol and acetic acid, advantageously $H_2O$ or phenol.

The electrolyte solutions are preferably aqueous solutions with a pH of between 6.5 and 7.5.

The supporting electrolyte of the invention comprises or consists of salts, in particular organic and inorganic salts and mixtures thereof. The salts may include buffers.

In a particular embodiment, the solvent of the anodic and cathodic electrolyte solution is identical. In this embodiment, the cathodic and anodic supporting electrolytes are identical or different. For instance, the cathodic supporting electrolyte is devoid of buffer, and the anodic supporting electrolyte comprises a phosphate buffer. The cathodic electrolyte may in particular consist of an aqueous solution of alkali metal salts, preferably chloride and carbonate salts, or mixtures thereof.

The anodic supporting electrolyte may comprise a buffer, preferably an alkali metal salt, and more preferably a mixture of $KH_2PO_4$ (monopotassium phosphate) and $K_2HPO_4$ (dipotassium phosphate) in an overall concentration of between 0.01 M and 1 M, more preferably of between 0.1 M and 0.6 M, and even more preferably of 0.4 M in the solvent.

In addition, in a preferred embodiment, $CO_2$ is present only in the cathodic compartment, and is thus partially dissolved in the cathodic electrolyte solution, while the anodic electrolyte solution is devoid of dissolved $CO_2$, as well as $HCO_3^-$ and $HCO_3^{2-}$ anions.

When production of pure CO in water is sought for, the pH of the cathodic and of the anodic electrolyte solution is preferably of between 6.5 and 7.5 in each compartment. Advantageously, the pH of the cathodic and of the anodic electrolyte solution is identical. In addition, the cathodic electrolyte solution is preferably devoid of any buffer. In particular, the cathodic electrolyte does not contain any phosphate buffer.

When production of syngas is sought for, the cathodic electrolyte may contain a buffer such as phosphate buffer.

In a particular embodiment, the catalyst is immobilized on at least one electrode (preferably the cathode) using a binder. Therefore, in this embodiment, at least one electrode (preferably the cathode) comprises a composition comprising the catalyst and a binder. Advantageously, the composition is coated as a film on the electrode.

Advantageously, the binder is selected from the group consisting of conductive polymers, ionomers and/or fluoropolymers preferably a fluorinated polymer with sulfonic end groups such as a perfluorosulfonic acid. Preferred examples of binders are ionomers such as polystyrene sulfonates, and in particular Nafion®, a sulfonated tetrafluoroethylene based fluoropolymer-copolymer. This embodiment is of particular interest because of the complementary nature of the ionic charges of the complexes used in the invention (positively charged anilinium groups) and of the binder (negatively charged sulfonic groups). Advantageously, conductive materials are added to the binder to improve the conductivity of the system. Preferred examples of conductive materials are conductive carbon materials, and in particular carbon powder and carbon nanotubes.

Said binder ensures: 1) a good conductivity, as well as 2) a good permeation of protons, $CO_2$ and CO through the composition by means of porosity of the polymer and/or the presence of acid-base functional groups within the polymer structure.

In another particular embodiment, the catalyst of the invention is used as a homogenous catalyst. In this case, the iron complex of the porphyrin of formula (I), as defined above, is in a concentration, in the electrolyte solution, of between 0.0001 and 0.01 M, preferably 0.001 M.

*First embodiment : water as solvent of the electrolyte solutions*

In a first embodiment, the solvent of the cathodic and anodic electrolyte solutions is water.

Advantageously, in this embodiment, the cathodic supporting electrolyte is devoid of any buffer other than carbonate buffer. In particular, it is devoid of phosphate buffer.

For instance, the cathodic supporting electrolyte is devoid of buffer, and the anodic supporting electrolyte comprises a phosphate buffer. The cathodic electrolyte may in particular consist of an aqueous solution of alkali metal salts, preferably chloride and carbonate salts, or mixtures thereof. Advantageously, the cathodic supporting electrolyte

may consist of a mixture of KCl (potassium chloride), preferably in a concentration of between 0.001 M and 1M, more preferably of 0.1M and $KHCO_3$ (potassium bicarbonate), preferably in a concentration of between 0.01 and 1M, more preferably of between 0.1M and 0.5M in the solvent. The anodic supporting electrolyte may comprise a buffer, preferably an alkali metal salt, and more preferably a mixture of $KH_2PO_4$ (monopotassium phosphate) and $K_2HPO_4$ (dipotassium phosphate) in an overall concentration of between 0.01 M and 1 M, more preferably of between 0.1 M and 0.6 M, and even more preferably of 0.4 M in the solvent.

In this embodiment, the catalyst is preferably immobilized as described above, for instance on at least one electrode (preferably the cathode) using a binder, optionally comprising a conductive material as described above.

*Second embodiment : organic medium as solvent of the electrolyte solutions*

In a second embodiment, the solvent of the cathodic and anodic electrolyte solutions is an organic medium, such as DMF (dimethylformamide), ACN (acetonitrile), and aqueous mixtures thereof. Advantageously, the electrolyte solutions comprise DMF (dimethylformamide) or ACN (acetonitrile) as the solvent. The electrolyte solution may further contain salts as the supporting electrolyte, such as n-$NBu_4PF_6$ for example. The electrolyte solution may further contain additives such as n-$NBu_4$ oxalate (tetrabutylammonium oxalate) for instance.

Preferably, the electrolyte solutions further comprise a proton donor, advantageously with a pKa value in DMF of between 18 and 31, in particular selected from the group consisting of water ($H_2O$), trifluoroethanol, phenol and acetic acid, advantageously $H_2O$ or phenol. Advantageously, when a proton donor is used, said proton donor is used in a concentration of between 100 mM and 5M, preferably between 100 mM and 3M, even more preferably between 1M and 3M. In some embodiments, the electrolyte solutions are solutions of phenol or water in DMF, preferably 0.0-5.0 M solutions of water or phenol in DMF, more preferably 0.0-2.5 M solutions of water or phenol in DMF, even more preferably 1.0-2.0 M solutions of water or phenol in DMF, and may contain additives (in particular supporting electrolytes) such as salts.

Preferably, the solvent is DMF and the electrolyte solution (preferably the cathodic electrolyte solution, and optionally the anodic electrolyte solution) further comprises at least a proton donor, preferably selected from the group consisting of water ($H_2O$), trifluoroethanol, phenol and acetic acid, even more advantageously $H_2O$ or phenol.

In this second embodiment, the catalyst is preferably homogeneous, *i.e.* it is solubilized into the electrolyte solution, and it is preferably in a concentration of between 0.0001 and 0.01 M, preferably 0.001 M in the electrolyte solution.

The catalyst may however be immobilized as described above, for instance on at least one electrode (preferably the cathode) using a binder.

***Use of the Electrochemical Cell***

The present invention further concerns a method comprising performing the electrochemical reduction of $CO_2$ using the electrochemical cell of the present invention, thereby producing CO gas or syngas in water.

The potential applied to the cathode vs NHE is preferably of between -2.5 V and -0.5 V versus NHE. In this embodiment, the current density of the cathode is typically below 30 $A/m^2$, in particular below 5 $A/m^2$.

The method of the invention allows production of CO or syngas, depending on the reaction conditions (in particular depending on the pH of the electrolyte solutions, on the presence of buffer in the cathodic electrolyte solution, and on the potential applied to the cathode). The method of the invention produces only minimal amounts of undesired byproducts. No formation of formic acid or formate is observed, in particular when production of pure CO is sought for.

The faradic yield of CO is thus advantageously of between 80% and 100%. When CO is selectively produced, The faradic yield of CO is preferably of between 90% and 100% , more preferably of 95% and 100%, in particular depending on the potential applied to the cathode.

When syngas is produced, the faradaic yield of $H_2$ is typically of between 0.5% and 20%, in particular of between 1% and 10%, for instance 2% to 7%.

When a mixture of CO and $H_2$ (syngas) is produced, in particular the pH of the aqueous solution and the potential applied to the cathode are adjusted so as to tune the $CO/H_2$ molar ratio of the produced gas

*First embodiment : water as solvent of the electrolyte solutions*

In a first embodiment, the solvent of the cathodic and anodic electrolyte solutions is water.

When syngas is produced in this embodiment, the pH of the cathodic electrolyte solution (notably through appropriate choice of buffer) and the potential applied to the cathode may be adjusted so as to tune (or choose) the $CO/H_2$ molar ratio of the produced gas. In this case, the cathodic electrolyte solution preferably comprises a buffer such as a phosphate buffer. The pH of the cathodic electrolyte solution is also typically of between 6.5 and 7.5.

For producing pure CO, the pH of the cathodic electrolyte solution is preferably of between 6.5 and 7.5, and

the cathodic electrolyte solution is advantageously devoid of buffer, in particular it is devoid of phosphate buffer. In this embodiment, the pH of the cathodic electrolyte solution is advantageously adjusted by adding alkali metal salts, preferably hydroxide, carbonate or bicarbonate alkali metal salts, more preferably KOH or $KHCO_3$, even more preferably $KHCO_3$. Preferably, in this embodiment, the potential applied to the cathode vs NHE is of between -1.5 V and -0.7 V versus NHE such as - 1.25V and -1.0V, more preferably between -1.21 V and -1.05V, even more preferably of -1.1V. More preferably, in this embodiment, the potential applied to the cathode vs NHE is of between -1.15V and -0.75V, more preferably between -1.1 V and -0.85V, even more preferably of -0.96V. In this embodiment, the current density of the cathode is typically below 30 A/m$^2$, in particular below 1 A/m$^2$.

*Second embodiment : organic medium as solvent of the electrolyte solutions*

In a second embodiment, the solvent of the cathodic and anodic electrolyte solutions is an organic medium, such as DMF (dimethylformamide), ACN (acetonitrile), and aqueous mixtures thereof. Preferably, the electrolyte solutions further comprise a proton donor as described above.

Advantageously, the intensity applied to the cathode is between 2 and 5 A/m$^2$, more preferably between 2.5 and 4 A/m$^2$, even more preferably between 3 and 3.5 A/m$^2$.

Advantageously, the potential applied to the cathode is between -2.5 V and -0.5 V versus NHE, more advantageously between -2.0 V and -0.5 V versus NHE, more advantageously between -1.5 V and -0.8 V versus NHE, more advantageously between - 1.3 V and -0.9 V versus NHE, and the intensity applied to the cathode is advantageously between 2 and 5 A/m$^2$, more preferably between 2.5 and 4 A/m$^2$, even more preferably between 3 and 3.5 A/m$^2$.

## DESCRIPTION OF THE DRAWINGS

Figure 1 represents the synthetic schema of Fe-*o*-TMA as used in example 1.

Figure 2 represents cyclic voltammograms of the iron porphyrins Fe-*o*-TMA, Fe-*p*-TMA, Fe-*p*-PSULF, FeTPP, FeF5TPP, FeF10TPP and FeF20TPP (see below) in the potential domain of the catalytic $CO_2$ reduction wave in DMF + 0.1 M n-$Bu_4NPF_6$ + 0.1 M $H_2O$ + 3 M PhOH, at 0.1 V/s under 1 atmosphere of $CO_2$ The current, *i*, is normalized against the peak current of the reversible one-electron $Fe^{II}/Fe^{I}$ reversible wave, $i_p^0$ obtained at the same scan rate (0.1 V/s). The abscissa axis represents the potential applied to the cathode vs SHE (in V), and the ordinate axis represents the normalized current.

**FeTPP**

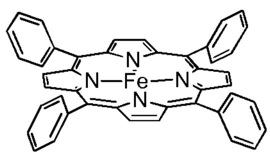

**F5FeTPP**

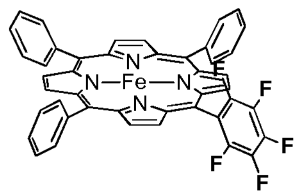

**F10FeTPP**

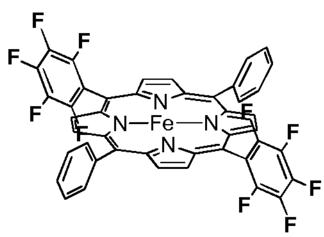

**F20FeTPP**

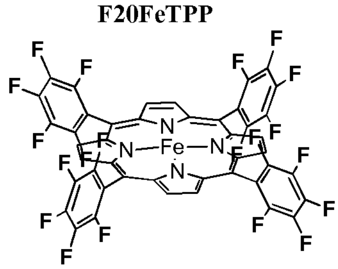

Figure 3. Cyclic voltammetry of Fe-*o*-TMA, Fe-*p*-TMA and Fe-*p*-PSULF (concentration: 1 mM) in DMF + 0.1 M *n*-$Bu_4NPF_6$ + 0.1 M $H_2O$ at 0.1 V/s under argon (grey) and under 1 atm. $CO_2$ in the presence of 3 M PhOH (black). The bottom figures are a blow-up of the upper figures showing the formation of CO on the reverse scan (see example

2). The current axis is normalized toward the peak current $i_p^0$ of a one-electron reversible wave at the same concentration at same scan rate (0.1 V/s) as can be obtained from the $Fe^{II/I}$ wave. The abscissa axis represents the potential applied to the cathode vs SHE (in V), and the ordinate axis represents the normalized current.

Figure 4. Elimination of the secondary phenomena by raising the scan rate (see Example 2). Cyclic voltammetry of the substituted iron porphyrins (conc.: 1 mM) in DMF + 0.1 M *n*-$Bu_4NPF6$ + 0.1 M $H_2O$ under 1 atm. $CO_2$ in the presence of 3M PhOH at scan rates (V/s), from bottom to top: Fe-*p*-TMA : 0.1, 1, 5, 10; Fe-*p*-PSULF: 0.1, 1, 2; Fe-*o*-TMA: 0.1, 0.5, 1, 2, 6, 10, 20, 30, 48, 96, 115. The current, *i*, is normalized against the peak current of the reversible one-electron $Fe^{II}/Fe^{I}$ reversible wave, $i_p^0$ obtained at 0.1 V/s. The abscissa axis represents the potential applied to the cathode vs SHE (in V), and the ordinate axis represents the normalized current.

Figure 5. a: Catalytic Tafel plots of Fe-*o*-TMA, Fe-*p*-TMA, Fe-*p*-PSULF, FeTPP, FeF5TPP, FeF10TPP and FeF20TPP. b: correlation between $TOF_{max} = k_{cat}$ and $E^0_{cat}$,

recalling the through-structure substituents effect and showing the coulombic interaction effects of positively and negatively charged substituents, including the huge gain in reactivity and overpotential brought about by positively charged substituents when located in ortho position of the phenyl ring.

Figure 6. depicts the benchmarking of all catalysts based on catalytic Tafel plots derived from cyclic voltammetry experiments in DMF or acetonitrile (see Costentin et al Proc. Natl. Acad. Sci. U.S.A. 2014, 111, 14990-14994 for details and references). The abscissa axis represents the overpotential $\eta$ (in Volts), and the ordinate axis represents log(TOF). See example 3 for experimental details.

a: example 3; b: in the presence of 3M PhOH, c: see structure above.

py = pyridine, tpy = 2,2':6',2"-terpyridine, bpy =2,2'-bipyridine, Mebimpy = 2,6-bis(1-methyl benzimidazol-2-yl)pyridine

**N2**

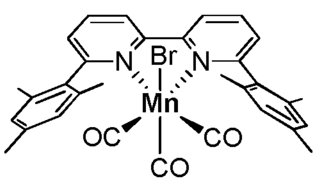

***m*-(triphos)$_2$Pd$_2$**

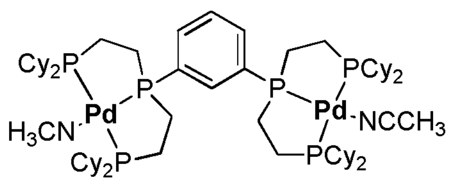

The vertical arrows indicate the overpotential values at which the electrolysis were carried out (see text and figure 8).

Figure 7. Preparative-scale electrochemical $CO_2$-to-CO conversion catalyzed by Fe-*p*-TMA (at an overpotential of 450 mV, vertical arrow in figure 7) and Fe-*o*-TMA (at an overpotential of 220 mV, vertical arrow in figure 7) in DMF + 0.1 M *n*-$Bu_4NPF_6$ + 0.1 M $H_2O$ under 1 atm. $CO_2$ in the presence of 3M PhOH (catalyst conc.: 0.5 mM). CO (full circle) and $H_2$ (open circle) faradaic yields (top) and CO-vs-$H_2$ selectivity (bottom), defined as: %CO/(%CO+% $H_2$).

Figure 8. Long run (84 hours) preparative-scale electrochemical $CO_2$-to-CO conversion catalyzed by Fe-o-TMA (at an overpotential of 220 mV, vertical arrow in figure 7) in DMF + 0.1 M *n*-$Bu_4NPF_6$ + 0.1 M $H_2O$ under 1 atm. $CO_2$ in the presence of 3 M PhOH (catalyst concentration: 0.5 mM). a. Charge evolution and b. Faradaic yields (CO: full symbols, $H_2$: open symbols) as a function of time during 24 h on a carbon electrode ( circles), then 18 h on a mercury electrode (squares), then a second 18 h period again on a mercury electrode (diamonds) and finally another 24 h period again on a carbon electrode (triangles).

a. The abscissa axis represents the time (in hours), and the ordinate axis represents the charged passed (in Coulomb).

b. The abscissa axis represents the time (in hours), and the ordinate axis represents the faradaic yield (in %).

Figure 9. Cyclic voltammetry of Fe-*p*-TMA, and Fe-p-TMA (concentration: 0.5 mM) in $H_2O$ + 0.1 M KCl at 0.1 V/s under argon (grey) and under 1 atm. $CO_2$ + 0.5M $KHCO_3$ (black) at pH 7.1. The bottom figures are a blow-up of the upper figures. The abscissa axis represents the potential applied to the cathode vs SHE (in V), and the ordinate axis represents the current (in $\mu$A).

Figure 10. Long run (60 hours) preparative-scale electrochemical $CO_2$-to-CO conversion catalyzed by Fe-o-TMA (at an applied potential of -0.86V, -0.91V and -0.96V vs. NHE) in $H_2O$ + 0.1 KCl + 0.5M $KHCO_3$ under 1 atm. $CO_2$ (catalyst concentration: 0.5 mM). Left ordinate axis represents the charge evolution (in Coulomb) and right ordinate axis represents the selectivity for CO (black squares) and $H_2$ (open circles) (in %) as a function of time during 60 h on a carbon electrode.

Figure 11. Preparative-scale electrochemical CO2-to-CO conversion catalyzed by Fe-*p*-TMA (at an applied potential

of -0.96V vs. NHE, grey line) and Fe-*o*-TMA (at an applied potential of -086V, -0.91V and -0.96V vs. NHE, black line) in $H_2O$ + 0.1 KCl + 0.5M $KHCO_3$ under 1 atm. $CO_2$ (catalyst concentration: 0.5 mM). CO (red dotsfull circle) and $H_2$ (blue open dotscircle) faradaic yields (top) and CO-vs-$H_2$ selectivity (bottom), defined as: %CO/(%CO+% $H_2$). The abscissa axis represents the time (in hours), and the ordinate axis represents the current (in mA).

## EXAMPLES

The following examples, while relating to particular embodiments of the invention, are intended for purposes of illustration only and are not intended to limit the scope of the invention.

As used herein, "$TOF_0$" represents the TurnOver Frequency at zero overpotential. The value of $TOF_0$ is obtained from extrapolation of the TOF vs. overpotential curve at zero overpotential. The TOF vs. overpotential curve is obtained from the experimental measurement of the current density (*I*) as function of potential (*E*) using cyclic voltammetry. For example, in the case of a simple mechanism (i.e. if the chemical steps in the catalytic loop are equivalent to a single step characterized by an apparent catalytic constant) the following relationship can be used:

$$TOF = \frac{I}{F\sqrt{\frac{D}{k_{cat}}C_{cat}^{0}}}$$

with *D* being the diffusion coefficient of the catalyst, $C_{cat}^{0}$ being its concentration in solution and $k_{cat}$ the catalytic rate constant. The value of $TOF_0$ is preferably obtained from extrapolation of the TOF vs. overpotential curve at zero overpotential. Said TOF vs. overpotential curve is for instance obtained such as described in Costentin et al ChemElectroChem, 2014, 1, 1226-1236, or calculated as detailed in Costentin et al, Science 2012 338, 90.

### Example 1. Synthesis and Characterization of Fe-o-TMA

*Chemicals.* Dimethylformamide (Acros, >99.8%, extra dry over molecular sieves), the supporting electrolyte $NBu_4PF_6$ (Fluka, purriss.) were used as received. All starting materials were obtained from Sigma-Aldrich, Fluka, Acros and Alfa-Aesar, and were used without further purification. $CHCl_3$ and $CH_2Cl_2$ were distilled from calcium hydride and stored under an argon atmosphere.

*Materials.* $^1H$ NMR spectra were recorded on a Bruker Avance III 400 MHz spectrometer and were referenced to the resonances of the solvent used. The mass spectra were recorded on a MALDI 4800 TOF/TOF. The elemental analysis were performed by the Microanalysis Service of the Institut de Chimie des Substances Naturelles (ICSN - CNRS), Avenue de la Terrasse, 91198 Gif-sur-Yvette cedex, France.

*Porphyrins.*

Iron(III) 5,10,15,20-tetrakis(4'-N,N,N-trimethylanilinium)-porphyrin pentachloride (**Fe-*p*-TMA**) was prepared as described elsewhere in Costentin et al. (Proc. Natl. Acad. Sci. U.S.A., 2015, 112, 6882).

Chloro iron(III) 5,10,15,20-tetrakis(4-sulfonatophenyl)porphyrin (tetrabutylammonium salt form) (**Fe-*p*-PSULF**) was prepared *in situ* by the deprotonation of commercial Chloro iron(III) 5,10,15,20-tetrakis(4-sulfonatophenyl)porphyrin (acid form) (LivChem Logistic) with 4 eq. of TBAOH.

**Fe-*p*-TMA**

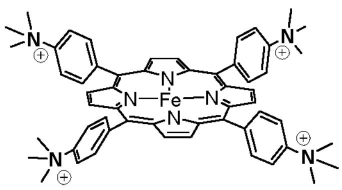

**Fe-*p*-PSULF**

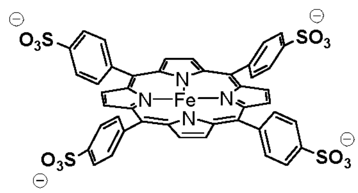

**Fe-*o*-TMA**

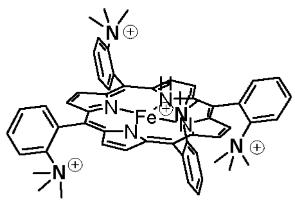

*Synthesis of Chloro iron(III) 5,10,15,20-tetra-(o-N,N,N-trimethylanilium)-porphyrin tetra trifluoromethanesulfonate (**Fe-o-TMA**).*

***5,10,15,20-tetra(o-nitrophenyl)-21H,23H-porphyrin. (1)***

Compound **1** was synthesized following a previously reported procedure by Collman et al. (J. Am. Chem. Soc. 1975, 97, 1427-1439).

Pyrrole (4.62mL; 66.17 mmol, 1eq) was added dropwise to a solution of 2-nitrobenzaldehyde (10 g, 66.2 mmol, leq.) in boiling acetic acid (200mL). After 20 min of stirring at reflux, the solution was cooled in an ice bath and chloroform (25 mL) was added. The mixture was filtered on a glass frit and the purple powder was thoroughly washed with chloroform and methanol until filtrates were colorless. The product was recovered and dried in the oven (1.8g, 13.6%)

***α,β,α,β-5,10,15,20-tetra(o-aminophenyl)-21H,23H-porphyrin. (2)***

Compound **2** was synthesized following a previously reported procedure by Collman et al. (J. Am. Chem. Soc. 1975, 97, 1427-1439).

Porphyrin **1** (1.8g; 2.265 mmol, 1eq.) was dissolved in concentrated HCl solution (60 mL). Excess $SnCl_2.2H_2O$ (7.67 g; 34 mmol, 15 eq.) was added, and the mixture quickly heated at 70°C for 25 min. After careful neutralization with ammonia, chloroform was added and the red mixture was stirred vigorously for 1h. The organic phase was kept, and the aqueous phase was extracted with chloroform 3 times. The organic phases were combined, filtered, reduced under vacuum, washed with dilute ammonia and water, dried on $Na_2SO_4$ and evaporated, affording a statistical mixture of the 4 atropisomers of the desired product with impurities (1.86g).

Pure *α,β,α,β-5,10,15,20-(o-aminophenyl)-21H,23H-porphyrin* can be obtained after purification by column chromatography on silica gel (gradient elution from $CH_2Cl_2/Et_2O$ = 100/0 to 95/5). The others atropisomers can be recovered after elution of $CH_2Cl_2$/MeOH 90/10. Refluxing the 3 others atropisomers in toluene for 4h affords a new statistical mixture of *5,10,15,20-(o-aminophenyl)-21H,23H-porphyrin.*

$^1$H NMR (400 MHz, $CDCl_3$) δ 8.91 (s, 8H,β-H), 7.88 (dd, *J* = 7.4, 1.4 Hz, 4H, Ar-H), 7.60 (td, *J* = 8.0, 1.5 Hz, 4H, Ar-H), 7.17 (td, *J* = 7.4, 1.1 Hz, 4H, Ar-H), 7.11 (dd, *J* = 8.1, 0.8 Hz, 4H, Ar-H), 3.51 (s, 8H, Ar-$NH_2$), -2.67 (s, 2H,Pyr-NH).

HR-MALDI-TOF ($[M+H^+]^+$) calculated for $C_{44}H_{35}N_8$: 675.2980, found: 675.3359

IR ($cm^{-1}$): 3464w, 3359w, 3317w, 2920w, 2855w, 1613m, 1491m, 1472m, 1449m, 1348w, 1297m, 1261w, 1213w, 1185w, 1158w, 965s, 800s, 741vs, 648s

***Chloro iron(III) α,β,α,β-5,10,15,20-tetra(o-aminophenyl)-porphyrin. (3)***

A solution of **2** (165 mg ; 0.245 mmol; leq.) and 2,6-lutidine (0.57mL ; 4.89mmol; 20 eq.) in freshly distilled THF (50 mL) is degassed with argon for 10 min. $FeBr_2$ (500 mg ; 1.69 mmol; 6.9 eq.) is added and the reaction mixture is stirred for 4 hours under argon. After evaporation *in vacuo,* the residue is solubilized in $CH_2Cl_2$ and washed with water (until aqueous phase gets colorless) and with saturated NaCl solution. The organic phase is then dried overs $MgSO_4$, filtered, evaporated and purified by column chromatography on silica gel (gradient elution from $CH_2Cl_2$/MeOH = 100/0 to 90/10) affording the desired product as a purple powder (185 mg) in 98% yield.

HR-MALDI-TOF ($[M-Cl^-]^+$) calculated for $C_{44}H_{32}FeN_8$: 728.2094, found: 728.1423 UV-vis (DMF, $\lambda_{max}$/nm, log(ε)/ $L.mol^{-1}.cm^{-1}$ ) : 414 (4.84), 576 (3.92), 626 (3.55)

IR ($cm^{-1}$) : 3464w, 3359w, 3204vw, 2916vw, 1612m, 1576m, 1495m, 1450m, 1334w, 1299m, 1267vw, 1201w, 1157w, 1067w, 997vs, 860w, 802m, 748s, 721m

***α,β,α,β-5,10,15,20-tetra(o-N,N-dimethylaminophenyl)-21H,23H-porphyrin (5)***

To a solution of porphyrin **2** (170mg; 0.25 mmol; 1eq.) in MeCN/MeOH (2/4 mL) is added formaldehyde (37% in $H_2O$; 750 μL; 10.1mmol; 40 eq.) and sodium cyanoborohydride (79.2mg; 1.26mmol; 5eq.). Glacial acetic acid (4.5

mL) is then poured dropwise and the reaction mixture is stirred for 4 hours at room temperature. The reaction mixture is diluted with $CH_2Cl_2$ (20mL) and cautiously washed with saturated $Na_2CO_3$ solution (2x30mL) and water (1x30mL). The organic phase is dried over $Na_2SO_4$, evaporated *in vacuo* and the residue is purified by column chromatography on silica gel (gradient elution from Petroleum ether/Ethyl acetate = 100/0 to 90/10) affording the desired product as a purple powder (112 mg) in 57% yield.

$^1$H NMR (400 MHz, $CDCl_3$) $\delta$ 8.75 (s, 8H, $\beta$-H), 7.99 (dd, *J* = 7.4, 1.6 Hz, 4H,Ar-H), 7.76 - 7.64 (m, 4H,Ar-H), 7.40 (d, *J* = 7.5 Hz, 4H,Ar-H), 7.30 (td, *J* = 7.4, 1.0 Hz, 4H,Ar-H), 2.23 (s, 24H, N-$CH_3$), -2.30 (s, 2H Pyr-NH).

$^{13}$C NMR (101 MHz, $CDCl_3$) $\delta$ 154.25 ($C_q$Ar-$NMe_2$), 137.66 ($CH_{Ar}$), 134.45 ($C_q$), 131.04* (CHp), 129.20 ($CH_{Ar}$), 120.06 ($CH_{Ar}$), 118.62 ($C_q$), 117.91 ($CH_{Ar}$), 43.55 ($N(CH_3)_2$) *. *Broad signal for $C_\beta$ and $C_\alpha$ could not be detected.

HR-MALDI-TOF ($[M+H^+]^+$) calculated for $C_{52}H_{51}N_8$: 787.4232, found: 787.4155 UV-vis (DMF, $\lambda_{max}$/nm, log($\varepsilon$)/L.mol$^{-1}$.cm$^{-1}$) : 429 (5.20), 527 (4.21), 563 (3.88), 603 (3.78), 660 (3.61)

IR (cm$^{-1}$): 3314vw, 2924w, 2855w, 2787w, 1714vw, 1591w, 1558w, 1493m, 1472m, 1449m, 1429m, 1345m, 1316w, 1217m, 1158m, 1098w, 1050m, 967s, 945s, 801vs, 758s, 724vs

***Chloro iron(III) $\alpha,\beta,\alpha,\beta$-5,10,15,20-tetra(o-N,N-dimethylaminophenyl)-porphyrin. (4)***

**Path A (see figure 1).** To a solution of porphyrin **3** (185mg; 0.24 mmol; 1eq.) in MeCN/$CH_2Cl_2$ (5/5 mL) is added formaldehyde (37% in $H_2O$; 721 $\mu$L; 9.69mmol; 40 eq.) and sodium cyanoborohydride (76.1mg; 1.21 mmol; 5eq.). Glacial acetic acid (5 mL) is then poured dropwise and the reaction mixture is stirred for 4 hours at room temperature. The reaction mixture is evaporated, the residue is solubilized in $CH_2Cl_2$ (30mL) and cautiously washed with saturated $Na_2CO_3$ solution (2x30mL), saturated NaCl solution (2x15mL) and water (1x20mL). The organic phase is dried over $MgSO_4$, filtered, evaporated *in vacuo* and the residue is purified by column chromatography on silica gel (gradient elution from $CH_2Cl_2$/MeOH = 100/0 to 95/5) affording the desired product as a purple powder (158 mg) in 75% yield.

**Path B.** A solution of **5** (30 mg ; 0.038 mmol; 1eq.) and 2,6-lutidine (89$\mu$L ; 0.76mmol; 20 eq.) in freshly distilled THF (8 mL) is degassed with argon for 10 min. $FeBr_2$ (158 mg ; 0.53 mmol; 14 eq.) is added and the reaction mixture is stirred for 20 hours at 40°C. Some remaining starting material could be observed by TLC, so more $FeBr_2$ (78 mg; 0.267 mmol; 7 eq.) are added and the reaction mixture is stirred for 5h at 45°C. After evaporation *in vacuo,* the residue is solubilized in $CH_2Cl_2$ and washed with water (until aqueous phase gets colorless) and with saturated NaCl solution. The organic phase is then dried over $MgSO_4$, filtered, evaporated and purified by column chromatography on silica gel ($CH_2Cl_2$/Ethyl Acetate 90/10 followed by $CH_2Cl_2$/MeOH 9/1) affording the desired product as a purple powder in 61% yield.

HR-MALDI-TOF ($[M-Cl]^+$) calculated for $C_{52}H_{48}FeN_8$: 840.3346 found: 840.2511 UV-vis (DMF, $\lambda_{max}$/nm, log($\varepsilon$)/L.mol$^{-1}$.cm$^{-1}$ ) : 426 (4.91), 583 (3.86), 631 (3.59), 703 (3.22)

IR (cm$^{-1}$) : 2929w, 2843w, 2782w, 1591w, 1494m, 1429m, 1330m, 1201w, 1156w, 1107w, 1065w, 1053w, 997vs, 946m, 801m, 757m

**Chloro Iron(III) 5,10,15,20-tetra(*o*-N,N,N-Trimethylanilinium)porphyrin tetra(trifluoromethanesulfonate) (Fe-*o*-TMA)**

To a solution of porphyrin **4** (40 mg ; 0.046 mmol; 1 eq.) in dry DMF (3 mL) under argon is added metyl trifluoromethanesulfonate (517$\mu$L ; 4.56 mmol; 100 eq.). The solution is stirred for 24h at 100°C under argon. Most of the DMF is evaporated under vacuum and the residual black waxy solid is solubilized in water (10mL) and washed with $CH_2Cl_2$ (4x20mL). The aqueous phase is evaporated and the brown solid is solubilized in water (5 mL) and purified by dialysis (membrane MWCO = 1000 g/mol) against deionised water (5x 90min). After removal of water, the residue is dried *in vacuo* for 24h affording a dark brown powder (65mg) in 92% yield.

ESI-MS : $[M+Cl^-]^{4+}$ calculated for $C_{56}H_{60}ClFeN_8$ : 233.8489; found: 233.8489.

UV-vis (DMF, $\lambda_{max}$/nm, log($\varepsilon$)/L.mol$^{-1}$.cm$^{-1}$) : 430 (4.90); 561 (3.81); 624 (3.43); 664 (3.28).

IR (cm$^{-1}$) : 3176w, 2827vw, 1471m, 1260vs (TfO), 1225vs (TfO), 1158vs (TfO), 1028vs (TfO), 948w, 834w, 759w, 666w, 634vs (TfO).

## Example 2. Cyclic Voltammetry and Electrolysis

**Methods and Instrumentation.**

*Cyclic Voltammetry.* The working electrode was a 3 mm-diameter glassy carbon (Tokai) disk carefully polished using decreasing size of diamond paste (from 15 to 1 $\mu$m), ultrasonically rinsed in absolute ethanol and dried before use or a mercury drop deposited on a 1 mm diameter gold disk. The counter-electrode was a platinum wire and the reference electrode was an aqueous SCE electrode. All experiments were carried out under argon or carbon dioxide atmosphere at 25 °C, the double-wall jacketed cell being thermostated by circulation of water. Cyclic voltamograms were

obtained by use of a Metrohm AUTOLAB instrument. Ohmic drop was compensated using the positive feedback compensation implemented in the instrument.

*Preparative Scale Electrolysis.* Electrolyzes were performed using a Princeton Applied Research (PARSTAT 2273) potentiostat. The experiments were carried out in a two-compartment cell with a glassy carbon crucible (the volume of the solution was 4 mL and active surface area was 12.7 $cm^2$) or a mercury pool (active surface area was 5.1 $cm^2$). The reference electrode was an aqueous SCE electrode and the counter electrode was a platinum wire in a bridge separated from the cathodic compartment by a glass frit, containing 0.2M $NEt_4CH_3CO_2$ + 0.1 M $NBu_4PF_6$ DMF solution. The electrolysis solution was purged with $CO_2$ during 20 min prior to electrolysis. Particular care was exerted to minimize the ohmic drop between working and reference electrodes. This was performed as follows: the reference electrode was directly immersed in the solution (without separated bridge) and put progressively closer to the working electrode until sustained oscillations appeared. It was then moved slightly away until the remaining oscillations were compatible with the catalytic current. The appearance of oscillations in this cell configuration does not require positive feedback compensation as it does with microelectrodes. The potentiostat + positive feedback compensation device system is equivalent to a self-inductance. Oscillations thus appear as soon as the resistance that is not compensated by the potentiostat comes close to zero as the reference electrode comes closer and closer to the working electrode surface.

Gas Detection. Gas chromatography analyses of gas evolved in the headspace during the electrolysis were performed with an Agilent Technologies 7820A GC system equipped with a thermal conductivity detector. CO and $H_2$ production was quantitatively detected using a CP-CarboPlot P7 capillary column (27.46 m in length and 25 $\mu$m internal diameter). Temperature was held at 150 °C for the detector and 34 °C for the oven. The carrier gas was argon flowing at 9.5 mL/min at constant pressure of 0.5 bars. Injection was performed via a 250-$\mu$L gas-tight (Hamilton) syringe previously degassed with $CO_2$. Conditions allowed detection of $H_2$, $O_2$, $N_2$, CO, and $CO_2$. Calibration curves for $H_2$ and CO were determined separately by injecting known quantities of pure gas. Results.

Figure 2 shows the $Fe^{I/0}$ cyclic voltammetric responses obtained under 1 atm $CO_2$ in the presence of 3 M phenol, with Fe-*o*-TMA, Fe-*p*-TMA, Fe-*p*-PSULF, FeTPP, FeF5TPP, FeF10TPP and FeF20TPP.

It immediately appears, before any treatment of the raw data, that the Fe-*o*-TMA is a far much better catalyst than any other molecules in the series both in terms of current and potential.

As seen in figure 3, the formation of CO is clearly attested by the observation of a cathodic shift of the $Fe^{I}/Fe^{II}$ reoxidation wave on the reverse scan in the cyclic voltammetric catalytic responses of Fe-*p*-TMA, Fe-*o*-TMA and Fe-*p*-PSULF. It matches what can be observed in the cyclic voltammetry of the $Fe^{II}/Fe^{I}$ couple in the presence of CO.

It is noticed (figures 2 and 3) that the current-potential responses of Fe-*p*-TMA, Fe-*o*-TMA and Fe-*p*-PSULF show peaks instead of the plateaus expected for fast catalytic processes. It is assumed that this is due to the interference of secondary phenomena such as substrate or co-substrate consumption, inhibition by product and possibly other phenomena that all increase with the charge passed. One way of fighting the interference of such phenomena is to raise the scan rate, and thereby, decrease the charge passed, so as to get back to a S-shaped current potential responses and derive the rate constant from the ensuing plateau current.

This is the treatment that has been applied to the raw cyclic voltammetric data as shown in figure 4. It is worth noting, en passant, that the scan rates required to reach an S-shaped CV responses are, as expected, the larger the stronger catalysis (in increasing order: Fe-*p*-PSULF, Fe-*p*-TMA, Fe-*o*-TMA).

Figure 7 shows the faradaic yields and the $CO/H_2$ selectivity factor for Fe-*o*-TMA (at an overpotential of 220 mV) and Fe-*p*-TMA (at an overpotential of 450 mV) over 7 hours electrolysis time. In both cases the CO-vs.-$H_2$ selectivity is excellent, even slightly better in the first case than in the second. During this seven-hour experiments both catalysts appear stable. Fe-o-TMA appears stable even during much longer electrolysis times, up to at least 84 hours as reported in figure 8 at an overpotential of 220 mV at which the TOF is $10^6$ $s^{-1}$.

## Example 3: Benchmarking of Fe-o-TMA with prior art complexes in organic medium:

Benchmarking with other catalysts in terms of overpotential and turnover frequency in water does not seem possible at the moment.

A comparison with the characteristics of other catalysts obtained in an aprotic solvent such as DMF or acetonitrile was thus made.

Combination of the foot-of-the wave analysis with increasing scan rates, which both minimize the effect of side-phenomena, allowed the determination of the turnover frequency as a function of the overpotential, leading to the "catalytic Tafel plot" for the Fe-o-TMA catalyst shown as the upper curve in figure 5. The turnover frequency (TOF), takes into account that the molecules that participate to catalysis are only those contained in the thin reaction-diffusion layer adjacent to the electrode surface in pure kinetic conditions. The overpotential, $\eta$, is the difference between the standard potential of the reaction to be catalyzed and the electrode potential. Correlations between TOF and $\eta$ provide catalytic Tafel plots that are able to benchmark the intrinsic properties of the catalyst independently of parameters such as cell configuration and size. Good catalysts stand in the upper left corner and bad catalysts in right bottom corner. These

plots allow one to trade between the rapidity of the catalytic reaction and the energy required to run it. The other Tafel plots shown in figure 6 are simply the repeat of what has been established in details in Costentin et al Proc. Natl. Acad. Sci. U.S.A. 2014, 111, 14990-14994.

The values of $k_{cat}$ required to establish the catalytic Tafel plots and the $k_{cat}$ vs. $E^0_{cat}$ for these three porphyrins may then be derived from the plateau current values, using the following equations. For the catalytic plateau current: $i_{pl} = FSC^0_{cat}\sqrt{D_{cat}}\sqrt{2k_{cat}}$, and for the one-electron diffusion current at 0.1 V/s: $i^0_p = FS \times 0.446 \times C^0_{cat}\sqrt{D_{cat}}\sqrt{\frac{Fv(=0.1\text{V/s})}{RT}}$. Using the ratio $\frac{i_{pl}}{i^0_p} = 2.24\sqrt{\frac{2k_{cat}}{0.1}\frac{RT}{F}}$ avoids determining S (electrode surface area), $C^0_{cat}$ and $D_{cat}$ (concentration and diffusion coefficient of the catalyst, respectively). Thus:

$$k_{cat} = \left(\frac{i_{pl,v}}{i^0_{p,0.1}}\right)^2 \frac{0.1}{2.24^2}\frac{F}{2RT}$$

The values of $k_{cat}$ thus obtained were then used to locate the catalytic Tafel plots of porphyrins (figure 5a) and to introduce the corresponding data points in the kcat vs. $E^0_{cat}$ correlation diagram (figure 5b).

A through-space effect clearly appears for Fe-*p*-TMA in so far that its representative point stands above the through-structure correlation line. That this effect results from the positive charges borne by the substituents is confirmed by the observation that the introduction of negative charges in similar positions produces the reverse effect (figure 6b). Although clearly present, these effect are necessarily small since the charges are rather distant from the reacting center, viz., the initial adduct between $CO_2$ and the iron(0) complex.

These observations encouraged us to introduce positive charged substituents closer to the reacting center in spite of the expected synthetic difficulties related to steric congestion of this type of substituted iron-porphyrin (see the Experimental Section below). The result - Fe-*o*-TMA - proved to be the best catalyst of the whole iron porphyrin series with considerable gains in terms of overpotential and of turnover frequency as can be seen in figure 5 and 6. The maximal turnover frequency is as high as $10^6$ $s^{-1}$ and the turnover frequency at zero overpotential larger than 300 $s^{-1}$.

The reason for this leap forward is most likely the stabilization of the initial iron(0) adduct by the interactions of the negative charge borne by the oxygens of $CO_2$ in this adduct with the nearby positive charges borne by the trimethylanilinium substituents on the porphyrin phenyls. The presence of phenol in large concentration then helps the proton-assisted reductive cleavage of one of the carbon-oxygen bond of $CO_2$. This is a particularly striking example of the power of close-distance through-space interactions in boosting of catalysis. Another type of close-distance through-space interactions as already been implemented to boost $CO_2$ reduction catalysis, albeit with a lesser efficiency, namely the introduction of ortho phenol groups in the porphyrin phenyls in which stabilization of the initial $Fe^0$ - $CO_2$ adduct is achieved by means of H-bonding. Comparison between the coulombic-interaction and H-bond-interaction series is shown in figure 6, as well as with the best examples that can be found in the literature. It again appears that Fe-*o*-TMA is the champion in all categories.

**Example 4: Catalysis in water**

Cyclic Voltammetry

The same experimental setup was used as in example 2.

The cyclic voltammetric response of Fe-p-TMA and Fe-o-TMA in water is reported on figure 9. Under Argon, Fe-o-TMA features two reversible waves attributed to the $Fe^{II}/Fe^{I}$ and $Fe^{I}/Fe^{0}$ couples while for Fe-p-TMA, only the $Fe^{II}/Fe^{I}$ couples appears as a reversible wave. This already indicates a poor reactivity of the $Fe^0$ state of Fe-*o*-TMA toward the proton reduction reaction.

Under $CO_2$, the two catalysts present a strong catalytic wave. The highest being the one of Fe-*o*-TMA. Furthermore, the onset of the catalytic wave of Fe-o-TMA is approximately 125mV more positive than the one of Fe-p-TMA.

For very efficient catalysts, the substrate diffusion can become the limiting factor of the catalysis. In this specific case of total catalysis, the peak current of the catalytic wave is independent of the kinetic constant and the catalyst concentration and can be calculated with the following equation:

| | |
|---|---|
| $i_p = 0.5 \text{ x } 2 \text{ x } FSC^0_{CO2}\sqrt{\frac{D_{CO2}Fv}{RT}}$<br><br>$i_p$ = 1.27mA | S = 0.07cm$^2$ (d=3mm)<br>$C^0_{CO2}$ = 30.10$^{-6}$ mol.cm$^{-3}$<br>$D_{CO2}$ =1. 10$^{-5}$ cm$^2$.s$^{-1}$<br>F=96500 C.mol$^{-1}$<br>V = 0.1 V/s<br>R=8.314 J.mol$^{-1}$.K$^{-1}$<br>T=293 K |

The experimental peak current for Fe-o-TMA is measured at 1.03 mA, which indicates that a total catalysis regime is almost reached.

Theses cyclic voltammetric experiments clearly indicate that Fe-o-TMA is a better catalyst than Fe-p-TMA both in term of kinetic rate and overpotential.

*Preparative-Scale Electrolysis in water.* Electrolyses were performed using a Princeton Applied Research PARSTAT 4000 potentiostat interfaced with VersaStudio software. The experiment was carried out in a two-compartment cell. The working electrode was a glassy carbon crucible (active surface area was 15cm$^2$). The reference electrode was an aqueous SCE electrode and the counter electrode a platinum grid in a bridge separated from the cathodic compartment by a glass frit. Ohmic drop was minimized by immersing directly the reference electrode into the solution as close as possible to the working electrode. The electrolysis cell was purged with $CO_2$ for 15 min before electrolysis then sealed for quantitative experiments or under a continuous flux for the long-time-scale electrolysis to avoid the $CO_2$ consumption. The cathodic electrolyte solution consisted in aqueous 0.5 mM Fe-*o*-TMA in 0.1M KCl and 0.5M $KHCO_3$ (pH 7.2) saturated with $CO_2$, while the anodic electrolyte solution consisted in an aqueous solution of phosphate buffer (0.2M) at pH 7.1.

*Gas Detection.* Gas chromatography analyses of gas evolved in the headspace during the electrolysis were performed with the same techniques than detailed in Example 3.

*Results*

| Catalyst | Potential (V vs.NHE) | Time | Current | Charge © | TON ($CO/H_2$) | Selectivity ($CO/H_2$) |
|---|---|---|---|---|---|---|
| Fe-*p*-TMA | -0.96V | 7h45 | ~360uA | 11.7 | 37,8/1.6 | 96/4 |
| Fe-*o*-TMA | -1.86V | 24h | ~800uA | 64.8 | 111/0 | 100/0 |
| Fe-*o*-TMA | -0.91V | 9h10 | 3 -> 2.6 mA | 92.8 | 159/0 | 100/0 |
| Fe-*o*-TMA | -0.91V | 8h40 | 3.4mA | 110 | 190/1 | 99.5/0.5 |
| Fe-*o*-TMA | -0.96V | 2h35 + 2h55 + 6h13 + 2h50 + 3h15 | 14 -> 2.7mA | 86.3+ 71.6 + 121.1 + 62.4 + 44.29 | 660/6.25 | 99/1 |

A series of preparative scale electrolyses were performed at three different applied potentials (-0.86V, -0.91V and -0.96V vs. NHE) over a total of 60h (figure 10). During the whole experiment, the system remained very selective towards $CO_2$ reduction with a faradaic efficiency above 99% for CO. The almost linear evolution of the charge with time indicates a quite good stability of the catalyst.

*Comparison with* Fe-*p*-TMA

A control electrolysis of 7h45 at -0.96V vs. NHE has been performed with Fe-*p*-TMA, with the same setup used for Fe-o-TMA, The measured current density was more than an order of magnitude lower for Fe-p-TMA compared to Fe-o-TMA (figure 11). Moreover, the selectivity was slightly lower for Fe-p-TMA (96% selectivity for CO, versus >99% for Fe-o-TMA).

**Summary**

Without wishing to be bound by theory, it may be concluded that through-spacesubstituent effects on the catalysis of the electrochemical $CO_2$-to-CO conversion by iron(0)-tetraphenyl porphyrins has been first investigated and evidenced by the introduction of four positively charged trimethylanilinium groups in para-position of the TPP phenyls (Fe-*p*-TMA). The assignment of this catalysis boosting effect to the coulombic interaction of these positive charges with the negative charges borne by the initial $Fe^0$-$CO_2$ adduct has been further confirmed by the negative catalytic effect observed when the four positive charges in Fe-*p*-TMA are replaced by four negative charges borne by sulfonate groups installed in the para-position of the TPP phenyls (Fe-*p*-PSULF). Optimization of the catalysis by means of coulombic stabilization of the initial $Fe^0$ - $CO_2$ adduct was reached when four positively charged trimethylanilinium groups are introduced in the ortho position of the TPP phenyls (Fe-*o*-TMA). The exceptional efficiency of the resulting catalyst is unprecedented with maximal turnover frequency as high as 106 $s^{-1}$ and is reached at a low overpotential of 220 mV. The selectivity for CO production is close to 100% while the catalyst appears extremely stable upon long term electrolysis, with no significant alteration for more than three and a half days.

**Claims**

1. Iron complex of a porphyrin of formula (I):

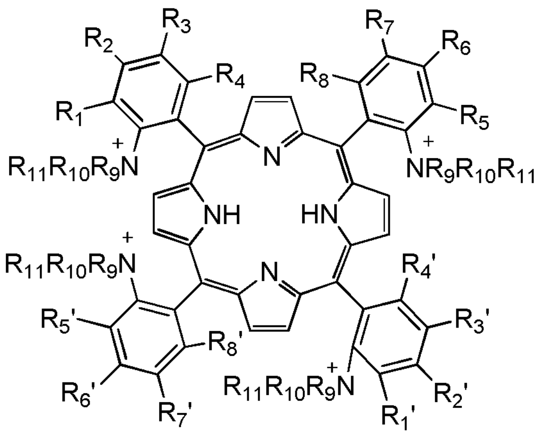

(I)

wherein $R_1$ to Rs and $R_1$' to $R_8$' are independently selected from the group consisting of H, OH, F and $C_1$-$C_6$-alkyl, provided that at least 2 of $R_1$-$R_4$ are H, at least 2 of $R_{1'}$-$R_{4'}$ are H, at least 2 of $R_5$-$R_8$ are H, and at least 2 of $R_{5'}$-$R_{8'}$ are H, and
$R_9$, $R_{10}$ and $R_{11}$ are independently selected from a $C_1$-$C_4$-alkyl group,

or atropisomers thereof,
and salts thereof.

2. The iron complex of claim 1, wherein $R_9$, $R_{10}$ and $R_{11}$ are a methyl group.

3. The iron complex of claim 1 or 2, wherein $R_1$ to $R_8$ and $R_1$' to $R_8$' are selected from the group consisting of H and $C_1$-$C_6$-alkyl.

4. The iron complex of any of claims 1 to 3, wherein the porphyrin of the invention is of formula (Ia):

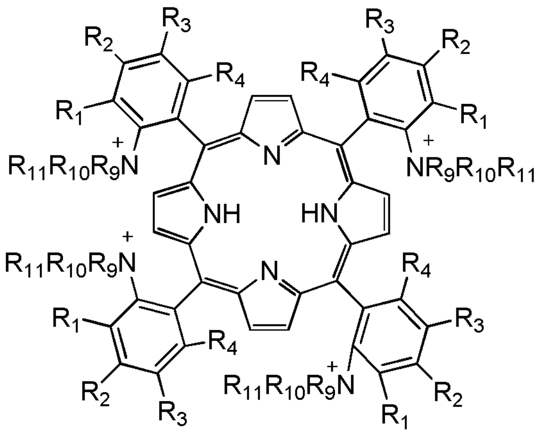

(Ia)

with $R_1$ to $R_4$ and $R_9$, $R_{10}$ and $R_{11}$ as defined in any of claims 1 to 3, or atropisomers thereof, and salts thereof.

**5.** The iron complex of any of claims 1 to 4, wherein the porphyrin of the invention is of formula (Ib) :

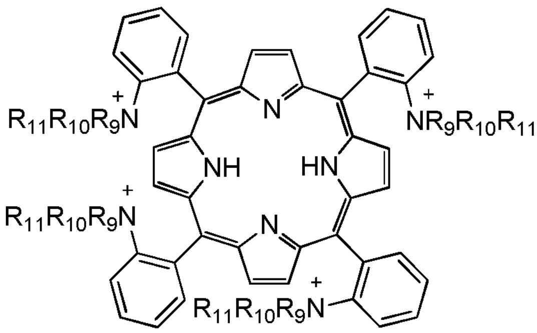

(Ib),

with $R_9$, $R_{10}$ and $R_{11}$ as defined in any of claims 1 to 3, or atropisomers thereof, and salts thereof.

**6.** The iron complex of claim 5, wherein the porphyrin of the invention is :

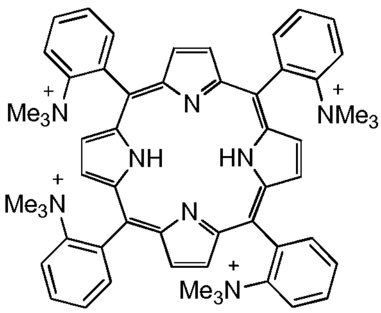

,

or atropisomers thereof, and salts thereof.

**7.** Use of the iron complex of any of claims 1-6, and salts thereof, as catalyst for the electrochemical reduction of $CO_2$ into CO with iron at the oxidation state of Fe(0).

**8.** The use of claim 7, wherein the iron complex is used as homogenous catalyst or is immobilized on at least one electrode using a binder, optionally containing conductive materials as additives.

**9.** A two-compartment electrochemical cell comprising at least:

- a cathodic compartment with a cathode and a cathodic electrolyte solution comprising a cathodic solvent and a cathodic supporting electrolyte, and the substrate $CO_2$,
- an anodic compartment with an anode and an anodic electrolyte solution comprising a solvent and an anodic supporting electrolyte,
- a power supply providing the energy necessary to trigger the electrochemical reactions involving the substrate,

and further comprising the iron complex of any of claims 1-6.

**10.** The electrochemical cell of claim 9, wherein $CO_2$ gas is present only in the cathodic compartment and the $CO_2$ pressure in the cathodic compartment of the electrochemical cell is of between 1 bar and 30 bars.

**11.** The electrochemical cell of any of claims 9 to 10, wherein the solvent of the electrolyte solutions is selected from dimethylformamide, acetonitrile, water or mixtures thereof.

**12.** The electrochemical cell of claim 11, wherein, when the solvent is water, the cathodic supporting electrolyte is devoid of buffer, and the anodic supporting electrolyte comprises a phosphate buffer.

**13.** The electrochemical cell of claim 11 or 12, wherein, when the solvent comprises dimethylformamide and/or acetonitrile, said solvent further comprises a proton donor with a pKa value in DMF of between 18 and 31, in particular selected from the group consisting of water, trifluoroethanol, phenol and acetic acid.

**14.** Method of reducing electrochemically $CO_2$ into CO using the electrochemical cell of any of claims 11 to 13.

**15.** The method of claim 14, wherein, when a mixture of CO and $H_2$ (syngas) is produced, the pH of the aqueous solution and the potential applied to the cathode are adjusted so as to tune the $CO/H_2$ molar ratio of the produced gas.

**Patentansprüche**

**1.** Eisenkomplex eines Porphyrins der folgenden Formel (I):

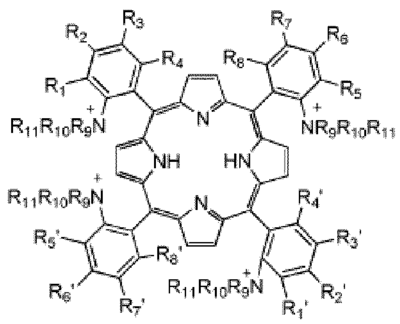

(I)

wobei $R_1$ bis $R_8$ und $R_1$' bis $R_8$' unabhängig aus der Gruppe bestehend aus H, OH, F und $C_1$-$C_6$-Alkyl ausgewählt sind,
vorausgesetzt, dass mindestens 2 von $R_1$ bis $R_4$ H sind, mindestens 2 von $R_1$' bis $R_4$' H sind, mindestens 2 von $R_5$ bis $R_8$ H sind, und mindestens 2 von $R_5$' bis $R_8$' H sind, und
$R_9$, $R_{10}$ und $R_{11}$ unabhängig aus einer $C_1$-$C_4$-Alkylgruppe ausgewählt sind,

oder Atropisomere davon
und Salze davon.

**2.** Eisenkomplex nach Anspruch 1, wobei $R_9$, $R_{10}$ und $R_{11}$ eine Methylgruppe sind.

**3.** Eisenkomplex nach Anspruch 1 oder 2, wobei $R_1$ bis $R_8$ und $R_1$' bis $R_8$' aus der Gruppe bestehend aus H und $C_1$-$C_6$-Alkyl ausgewählt sind.

**4.** Eisenkomplex nach einem der Ansprüche 1 bis 3, wobei das Porphyrin der Erfindung die folgende Formel (Ia)

aufweist:

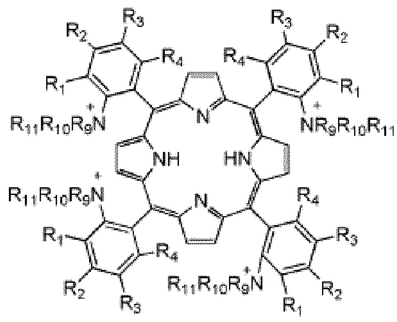

(Ia)

wobei $R_1$ bis $R_4$ sowie $R_9$, $R_{10}$ und $R_{11}$ wie in einem der Ansprüche 1 bis 3 definiert sind, oder Atropisomere davon und Salze davon.

**5.** Eisenkomplex nach einem der Ansprüche 1 bis 4, wobei das Porphyrin der Erfindung die folgende Formel (Ib) aufweist:

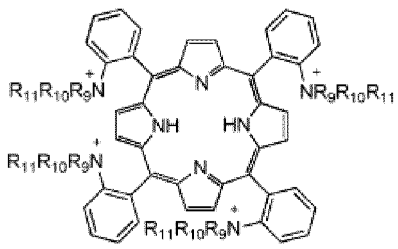

(Ib),

wobei $R_9$, $R_{10}$ und $R_{11}$ wie in einem der Ansprüche 1 bis 3 definiert sind, oder Atropisomere davon und Salze davon.

**6.** Eisenkomplex nach Anspruch 5, wobei das Porphyrin der Erfindung

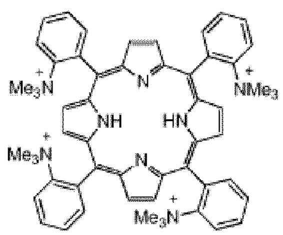

,

ist, oder Atropisomere davon und Salze davon.

**7.** Verwendung des Eisenkomplexes nach einem der Ansprüche 1 bis 6 und Salze davon als Katalysator für die elektrochemische Reduktion von $CO_2$ zu CO mit Eisen im Oxidationszustand von Fe(0).

**8.** Verwendung nach Anspruch 7, wobei der Eisenkomplex als homogener Katalysator verwendet oder unter Verwendung eines Bindemittels, das optional leitende Materialien als Additive umfasst, auf mindestens einer Elektrode immobilisiert wird.

**9.** Elektrochemische Zweikammerzelle, umfassend mindestens:

- eine Kathodenkammer mit einer Kathode und einer kathodischen Elektrolytlösung, die ein kathodisches Lösungsmittel und einen kathodischen Trägerelektrolyten umfasst, und dem Substrat $CO_2$,
- eine Anodenkammer mit einer Anode und einer anodischen Elektrolytlösung, die ein Lösungsmittel und einen

anodischen Trägerelektrolyten umfasst,
- eine Leistungsversorgung, welche die Energie bereitstellt, die zum Auslösen der elektrochemischen Reaktionen unter Einbeziehung des Substrats notwendig ist,

und ferner umfassend den Eisenkomplex nach einem der Ansprüche 1 bis 6.

**10.** Elektrochemische Zelle nach Anspruch 9, wobei $CO_2$-Gas nur in der Kathodenkammer vorhanden ist, und der $CO_2$-Druck in der Kathodenkammer der elektrochemischen Zelle 1 bar bis 30 bar beträgt.

**11.** Elektrochemische Zelle nach einem der Ansprüche 9 bis 10, wobei das Lösungsmittel der Elektrolytlösungen aus Dimethylformamid, Acetonitril, Wasser oder Mischungen davon ausgewählt ist.

**12.** Elektrochemische Zelle nach Anspruch 11, wobei, wenn das Lösungsmittel Wasser ist, der kathodische Trägerelektrolyt frei von Puffer ist, und der anodische Trägerelektrolyt einen Phosphatpuffer umfasst.

**13.** Elektrochemische Zelle nach Anspruch 11 oder 12, wobei, wenn das Lösungsmittel Dimethylformamid und/oder Acetonitril umfasst, das Lösungsmittel ferner einen Protonendonator mit einem pKa-Wert in DMF von 18 bis 31 umfasst und insbesondere aus der Gruppe bestehend aus Wasser, Trifluorethanol, Phenol und Essigsäure ausgewählt ist.

**14.** Verfahren zum elektrochemischen Reduzieren von $CO_2$ zu CO unter Verwendung der elektrochemischen Zelle nach einem der Ansprüche 11 bis 13.

**15.** Verfahren nach Anspruch 14, wobei, wenn ein Gemisch aus CO und $H_2$ (Synthesegas) erzeugt wird, der pH der wässrigen Lösung und das an die Kathode angelegte Potenzial angepasst werden, um das CO/$H_2$-Molverhältnis des erzeugten Gases abzustimmen.

## Revendications

**1.** Complexe de fer et d'une porphyrine de formule (I) :

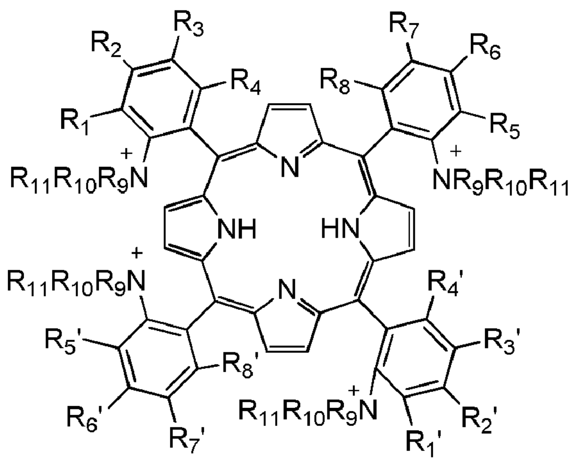

(I)

dans laquelle $R_1$ à $R_8$ et $R_1$' et $R_8$' sont indépendamment choisis dans l'ensemble constitué par H, OH, F et alkyle en $C_1$ à $C_6$,
sous réserve qu'au moins 2 parmi $R_1$ à $R_4$ soient H, au moins 2 parmi $R_1$' à $R_4$' soient H, au moins 2 parmi $R_8$ à $R_8$ soient H, et au moins 2 parmi $R_5$' à $R_8$' soient H, et
$R_9$, $R_{10}$ et $R_{11}$ sont indépendamment choisis parmi les groupes alkyle en $C_1$ à $C_4$,

ou ses atropisomères,
et ses sels.

**2.** Complexe de fer selon la revendication 1, dans lequel $R_9$, $R_{10}$ et $R_{11}$ sont des groupes méthyle.

3. Complexe de fer selon la revendication 1 ou 2, dans lequel $R_1$ à $R_8$ et $R_1'$ à $R_8'$ sont choisis dans l'ensemble constitué par H et alkyle en $C_1$ à $C_6$.

4. Complexe de fer selon l'une quelconque des revendications 1 à 3, dans lequel la porphyrine de l'invention est de formule (Ia) :

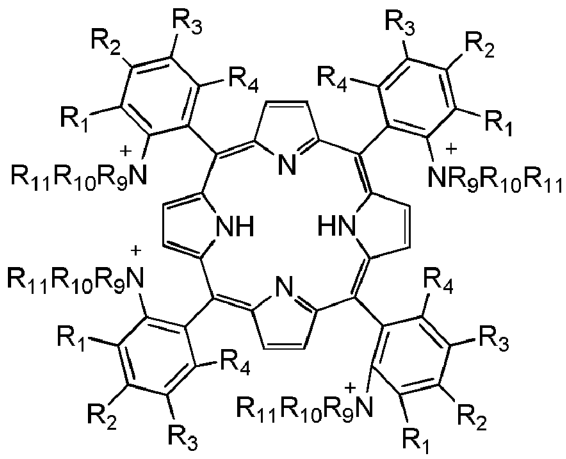

(Ia)

dans laquelle $R_1$ à $R_4$ et $R_9$, $R_{10}$ et $R_{11}$ sont tels que définis dans l'une quelconque des revendications 1 à 3, ou ses atropisomères, et ses sels.

5. Complexe de fer selon l'une quelconque des revendications 1 à 4, dans lequel la porphyrine de l'invention est de formule (Ib) :

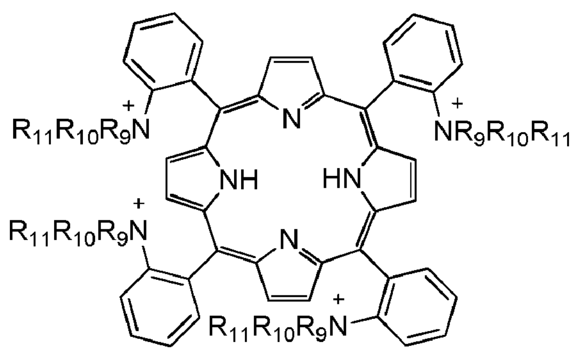

(Ib),

dans laquelle $R_9$, $R_{10}$ et $R_{11}$ sont tels que définis dans l'une quelconque des revendications 1 à 3, ou ses atropisomères, et ses sels.

6. Complexe de fer selon la revendication 5, dans lequel la porphyrine de l'invention est :

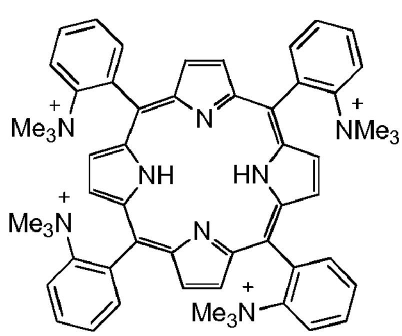

,

ou ses atropisomères, et ses sels.

**7.** Utilisation du complexe de fer de l'une quelconque des revendications 1 à 6, et de ses sels, en tant que catalyseur pour la réduction électrochimique de $CO_2$ en CO avec du fer dans l'état d'oxydation Fe(0).

**8.** Utilisation selon la revendication 7, dans laquelle le complexe de fer est utilisé en tant que catalyseur homogène ou est immobilisé sur au moins une électrode par utilisation d'un liant, contenant éventuellement des matériaux conducteurs en tant qu'additifs.

**9.** Cellule électrochimique à deux compartiments comprenant au moins :

- un compartiment cathodique avec une cathode et une solution d'électrolyte cathodique comprenant un solvant cathodique et un électrolyte de support cathodique, et le substrat $CO_2$,
- un compartiment anodique avec une anode et une solution d'électrolyte anodique comprenant un solvant et un électrolyte de support anodique,
- une source d'alimentation apportant l'énergie nécessaire pour déclencher les réactions électrochimiques impliquant le substrat,

et comprenant en outre le complexe de fer de l'une quelconque des revendications 1 à 6.

**10.** Cellule électrochimique selon la revendication 9, dans laquelle du $CO_2$ gazeux est présent uniquement dans le compartiment cathodique et la pression du $CO_2$ dans le compartiment cathodique de la cellule électrochimique est comprise entre 1 bar et 30 bar.

**11.** Cellule électrochimique selon l'une quelconque des revendications 9 et 10, dans laquelle le solvant des solutions d'électrolyte est choisi parmi le diméthylformamide, l'acétonitrile, l'eau, et leurs mélanges.

**12.** Cellule électrochimique selon la revendication 11, dans laquelle, quand le solvant est l'eau, l'électrolyte de support cathodique est exempt de tampon, et l'électrolyte de support anodique comprend un tampon phosphate.

**13.** Cellule électrochimique selon la revendication 11 ou 12, dans laquelle quand le solvant comprend du diméthylformamide et/ou de l'acétonitrile, ledit solvant comprend en outre un donneur de protons ayant une valeur de pKa dans le DMF comprise entre 18 et 31, en particulier choisi dans l'ensemble constitué par l'eau, le trifluoroéthanol, le phénol, et l'acide acétique.

**14.** Procédé pour réduire électrochimiquement du $CO_2$ en CO, utilisant la cellule électrochimique de l'une quelconque des revendications 11 à 13.

**15.** Procédé selon la revendication 14, dans lequel, quand un mélange de CO et de $H_2$ (gaz de synthèse) est produit, le pH de la solution aqueuse et le potentiel appliqué à la cathode sont ajustés de façon que le rapport molaire $CO/H_2$ du gaz produit soit accordé.

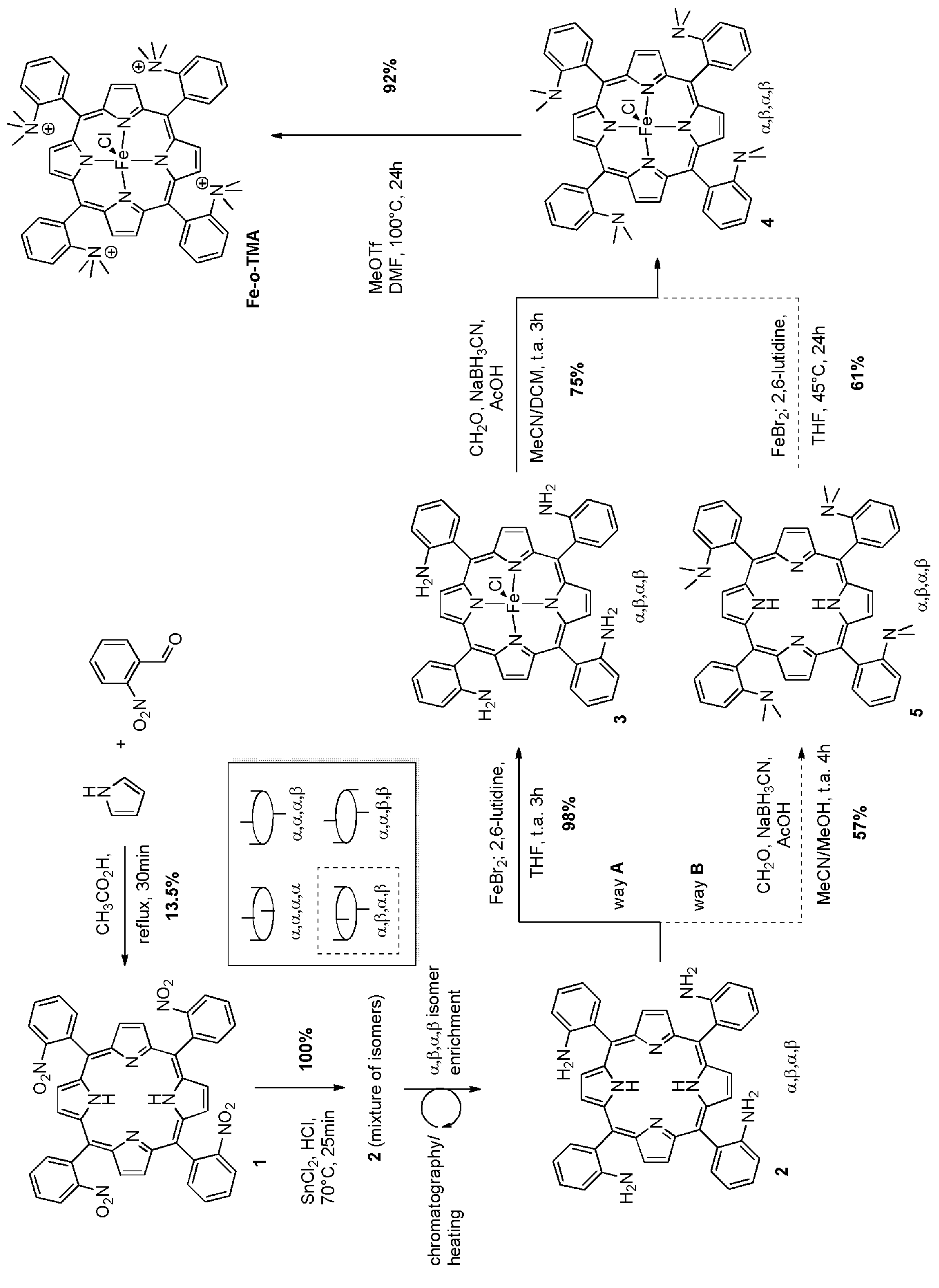
CH3CO2H,
reflux, 30min
13.5%
1
SnCl2, HCl,
70°C, 25min
100%
2 (mixture of isomers)
chromatography/
heating
α,β,α,β isomer
enrichment
α,α,α,α
α,α,α,β
α,β,α,β
α,α,β,β
2
α,β,α,β
way A
way B
FeBr2; 2,6-lutidine,
THF, t.a. 3h
98%
CH2O, NaBH3CN,
AcOH
MeCN/MeOH, t.a. 4h
57%
3
α,β,α,β
5
α,β,α,β
CH2O, NaBH3CN,
AcOH
MeCN/DCM, t.a. 3h
75%
FeBr2; 2,6-lutidine,
THF, 45°C, 24h
61%
4
α,β,α,β
MeOTf
DMF, 100°C, 24h
92%
Fe-o-TMA

**Figure 1**

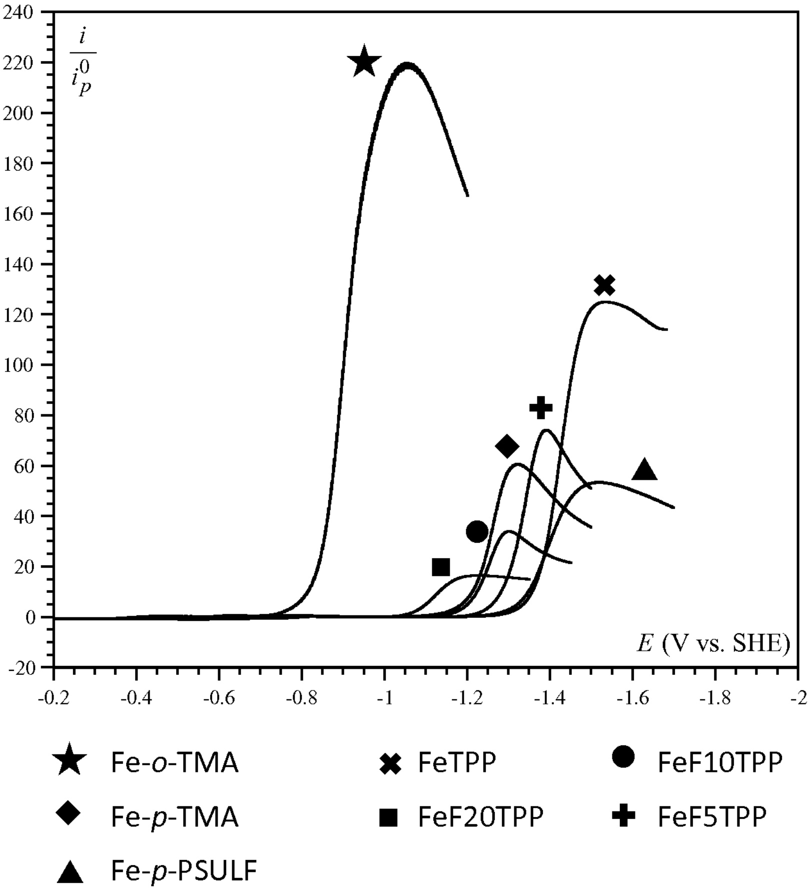

240
220
200
180
160
140
120
100
80
60
40
20
0
-20
i / i_p^0
E (V vs. SHE)
-0.2
-0.4
-0.6
-0.8
-1
-1.2
-1.4
-1.6
-1.8
-2
Fe-o-TMA
FeTPP
FeF10TPP
Fe-p-TMA
FeF20TPP
FeF5TPP
Fe-p-PSULF


**Figure 2**

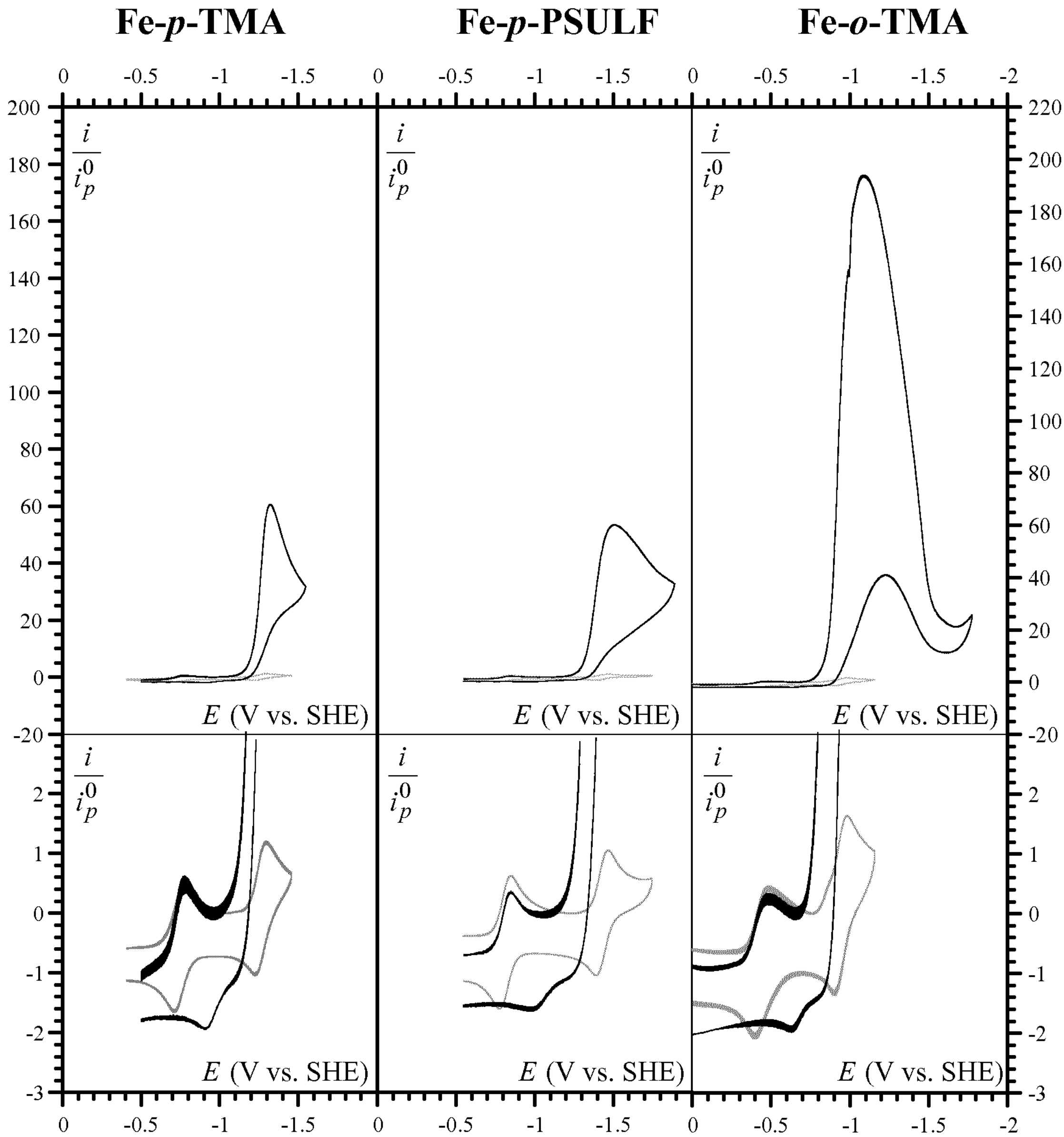
Fe-p-TMA
Fe-p-PSULF
Fe-o-TMA
0
-0.5
-1
-1.5
-2
200
180
160
140
120
100
80
60
40
20
0
-20
220
2
1
-1
-2
-3
i / i_p^0
E (V vs. SHE)

**Figure 3**

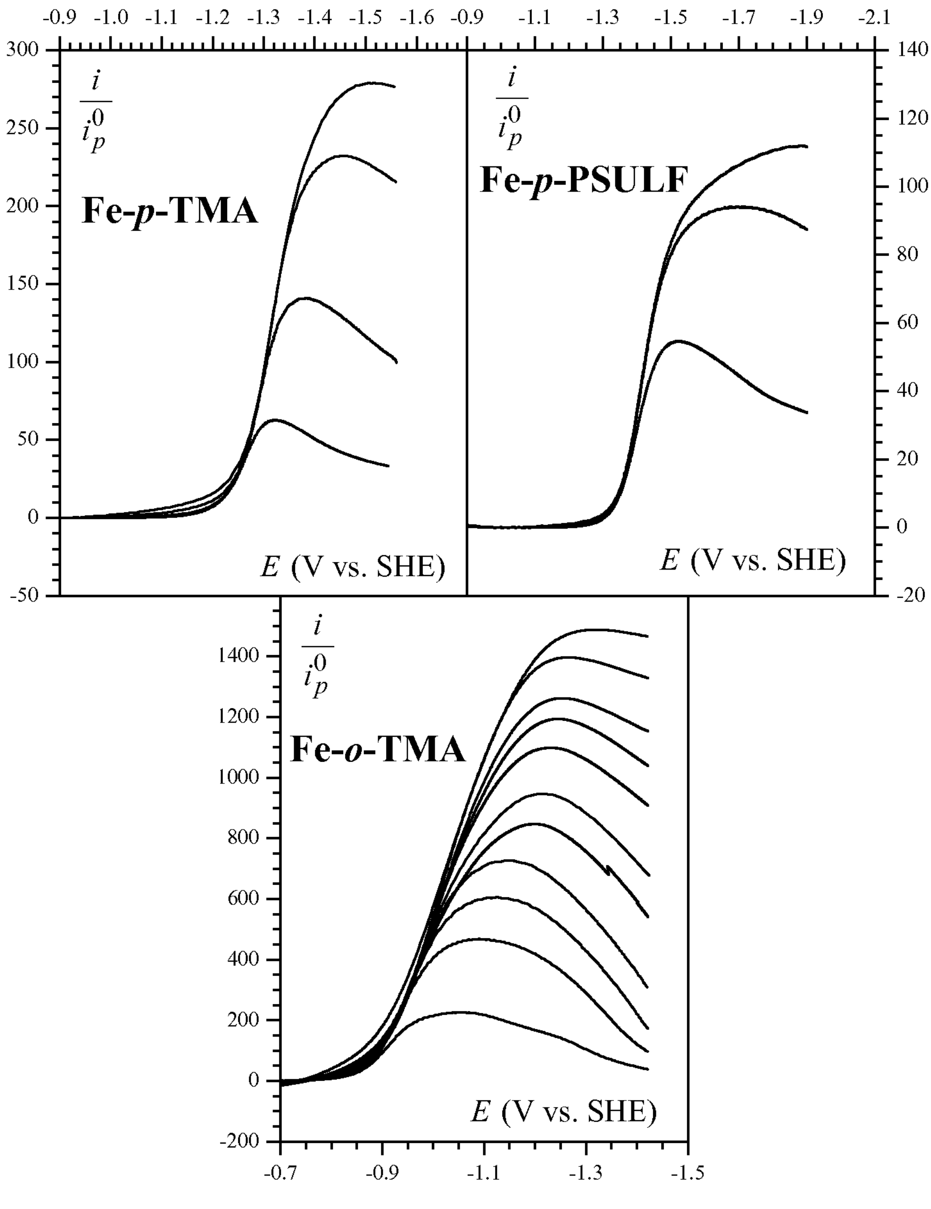
-0.9 -1.0 -1.1 -1.2 -1.3 -1.4 -1.5 -1.6
-0.9 -1.1 -1.3 -1.5 -1.7 -1.9 -2.1
300
250
200
150
100
50
0
-50
140
120
100
80
60
40
20
0
-20
Fe-p-TMA
Fe-p-PSULF
E (V vs. SHE)
E (V vs. SHE)
Fe-o-TMA
1400
1200
1000
800
600
400
200
0
-200
-0.7 -0.9 -1.1 -1.3 -1.5
E (V vs. SHE)

**Figure 4**

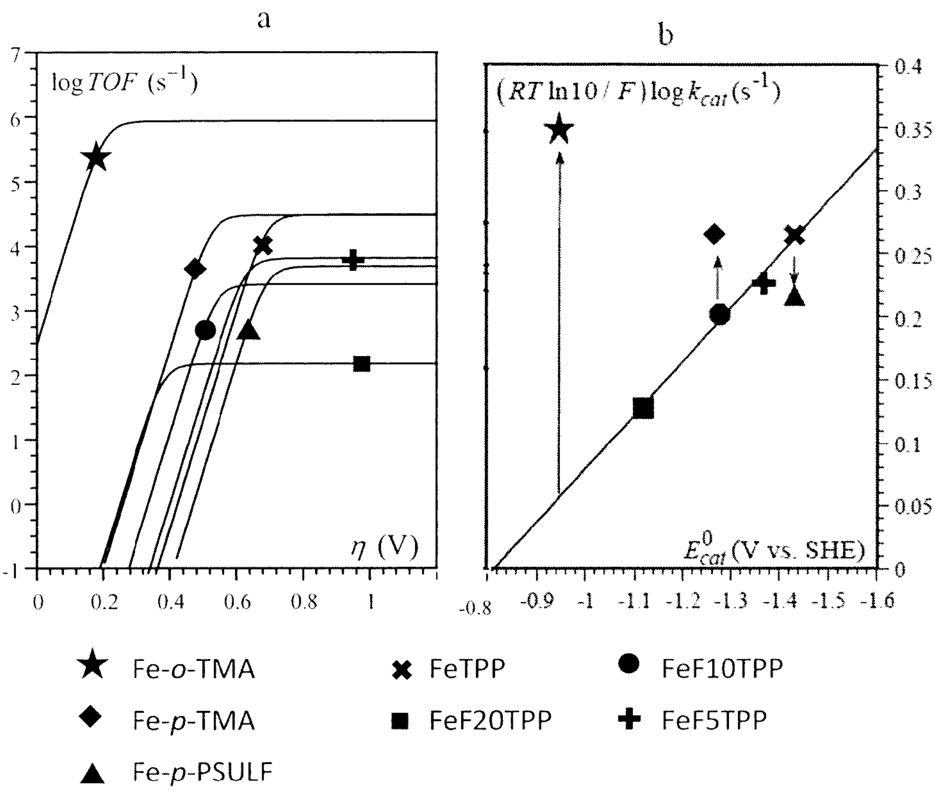


Figure 5

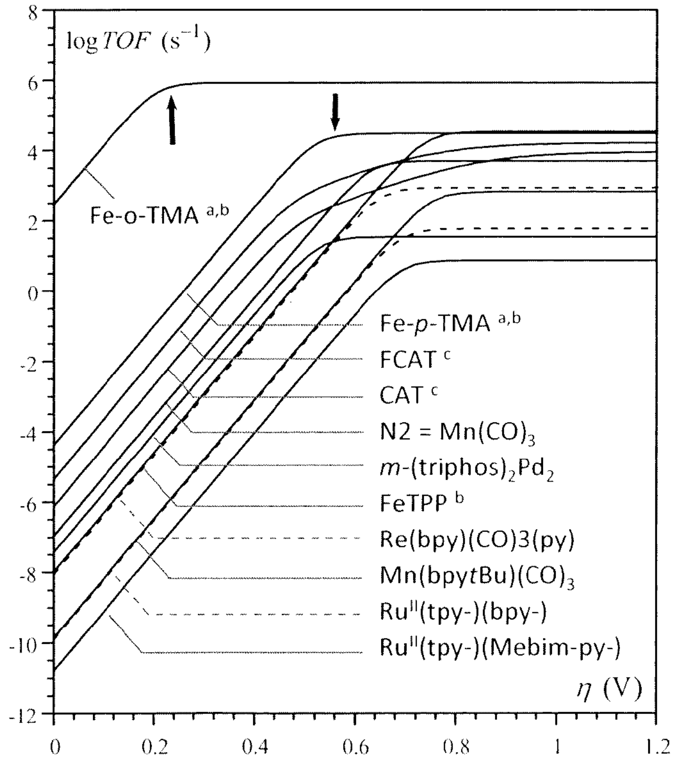


Figure 6

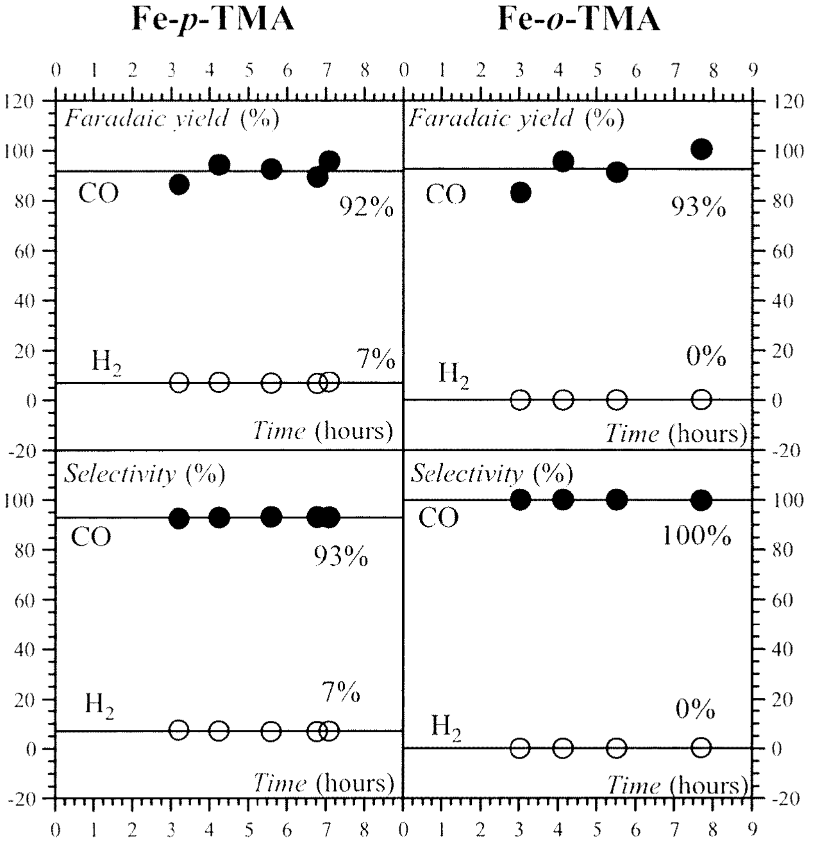


**Figure 7**

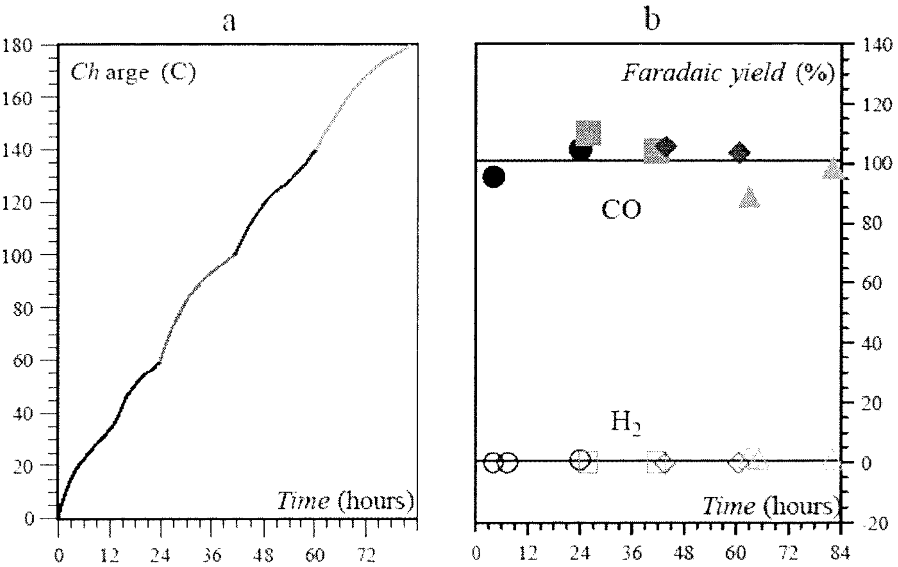


**Figure 8**

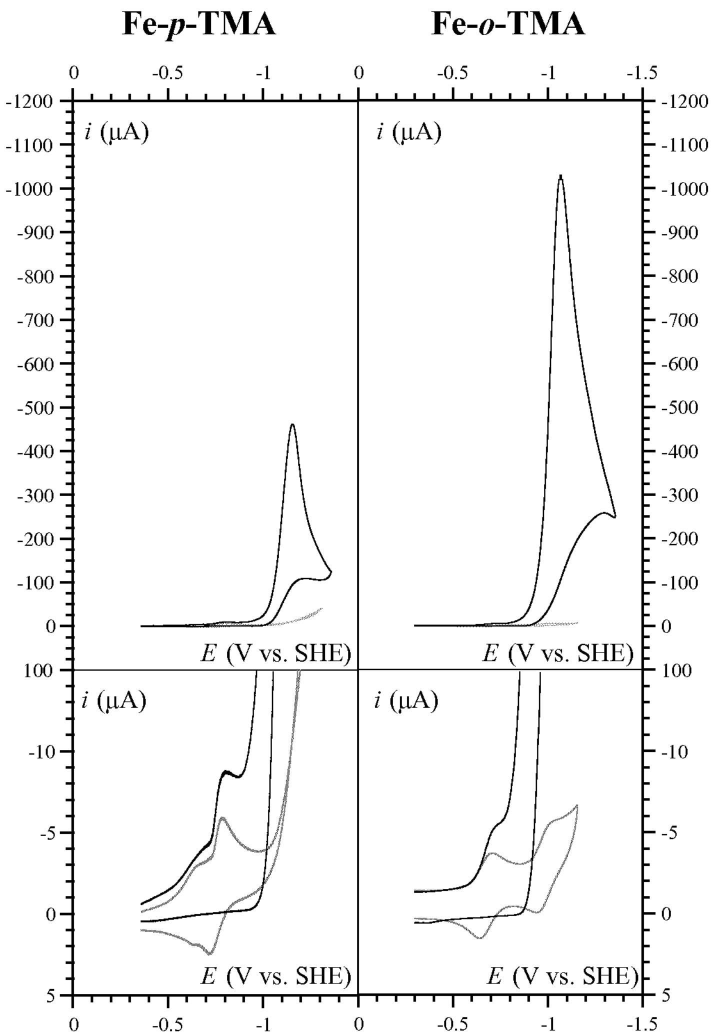
Fe-p-TMA
Fe-o-TMA
i (μA)
E (V vs. SHE)
0
-0.5
-1
-1.5
-1200
-1100
-1000
-900
-800
-700
-600
-500
-400
-300
-200
-100
100
-10
-5
5

**Figure 9**

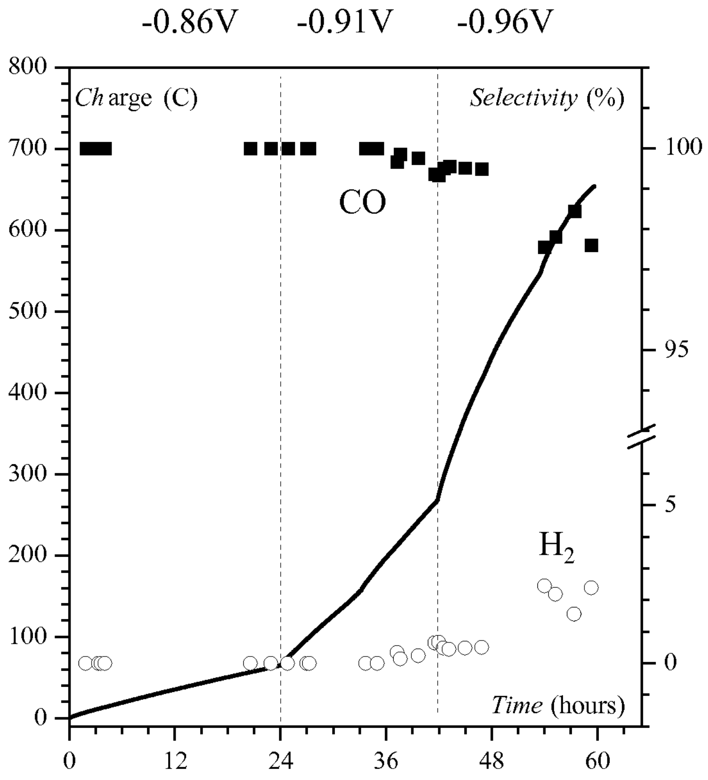


**Figure 10**

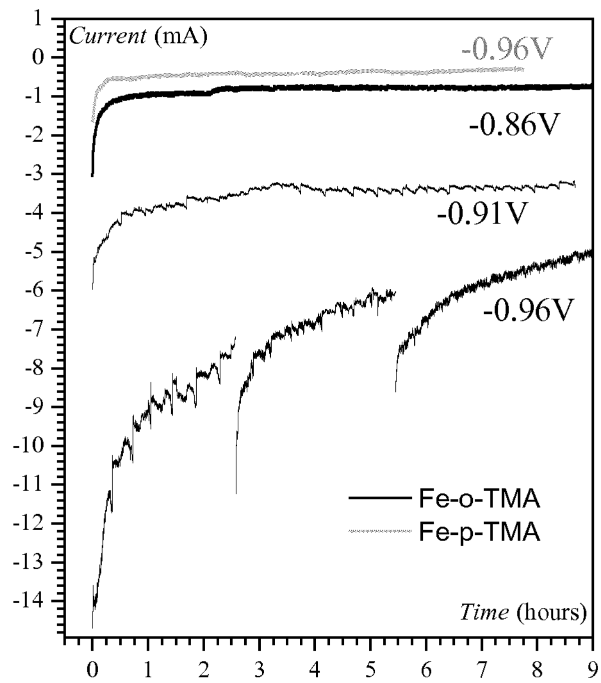


**Figure 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Chem. Soc. Rev.,* 2013, vol. 42, 2423
- *J. Am. Chem. Soc.,* 1996, vol. 118, 1769
- *J. Phys. Chem.,* 1996, vol. 100, 19981
- *J. Am. Chem. Soc.,* 2013, vol. 135, 9023
- *Science,* 2012, vol. 338, 90
- *Proc. Natl. Acad. Sci. U.S.A.,* 2014, vol. 111, 14990-14994
- **CAO et al.** *Acta Chimica Sinica,* 1986, vol. 44 (220), 133-139
- *Proc. Natl. Acad. Sci. U.S.A.,* 2015, vol. 112, 6882
- **COSTENTIN et al.** *Proc. Natl. Acad. Sci. U.S.A.,* 2014, vol. 111, 14990-14994
- **COSTENTIN et al.** *ChemElectroChem,* 2014, vol. 1, 1226-1236
- **COSTENTIN et al.** *Science,* 2012, vol. 338, 90
- **COSTENTIN et al.** *Proc. Natl. Acad. Sci. U.S.A.,* 2015, vol. 112, 6882
- **COLLMAN et al.** *J. Am. Chem. Soc.,* 1975, vol. 97, 1427-1439